(19) Europäisches Patentamt
European Patent Office
Office européen des brevets

(11) EP 4 644 904 A1

(12) **EUROPEAN PATENT APPLICATION**
published in accordance with Art. 153(4) EPC

(43) Date of publication:
**05.11.2025 Bulletin 2025/45**

(21) Application number: **23910644.6**

(22) Date of filing: **26.12.2023**

(51) International Patent Classification (IPC):
***G01N 33/72*** *(2006.01)* ***G01N 33/53*** *(2006.01)*
***C12Q 1/28*** *(2006.01)*

(52) Cooperative Patent Classification (CPC):
**C12Q 1/28; G01N 33/53; G01N 33/72**

(86) International application number:
**PCT/CN2023/142070**

(87) International publication number:
**WO 2024/140721 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 CN 202211691520**

(71) Applicant: **Shenzhen Mindray Bio-Medical Electronics Co., Ltd.**
**Shenzhen, Guangdong 518057 (CN)**

(72) Inventors:
- **WANG, Hong**
  **Shenzhen, Guangdong 518057 (CN)**
- **JIANG, Ming**
  **Shenzhen, Guangdong 518057 (CN)**

(74) Representative: **KIPA AB**
**Drottninggatan 11**
**252 21 Helsingborg (SE)**

(54) **REAGENT FOR DETECTING GLYCATED HEMOGLOBIN, DETECTION METHOD, AND USE**

(57) A reagent for detecting glycated hemoglobin, a detection method, and use. The reagent for detecting glycated hemoglobin by using a turbidimetric method comprises at least one amphiphilic compound selected from compound A and compound B. Compound A is a compound represented by the following formula A: $R_1$ being selected from a C10-C20 alkyl and $-(CH_2)_{1-3}$-N(H)C(O)-$R_3$, and $R_3$ being selected from a C8-C14 alkyl; and $R_2$ being selected from $-(CH_2)_{1-3}COO^-$ and $-(CH_2)_{1-3}SO_3^-$. Compound B is a copolymer of an olefin monomer containing a phosphorylcholine group and an acryl or methacryloyl monomer. The reagent can be used to test blood samples having a hemoglobin content in a range at or below 300 g/L, especially in a low value range of 60 g/L, and obtain accurate glycated hemoglobin detection results.

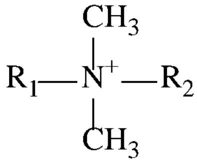

(A)

EP 4 644 904 A1

## Description

CROSS-REFERENCE

The present application refers to Chinese Patent Application No. 202211691520.2, filed on December 27, 2022, entitled "REAGENT AND KIT FOR DETECTING GLYCATED HEMOGLOBIN, DETECTION METHOD, AND USE," which is incorporated herein by reference in its entirety.

TECHNICAL FIELD

The present disclosure relates to the field of blood analysis, and in particular to a reagent for detecting glycated hemoglobin, a detection method, and use.

BACKGROUND

Glycated hemoglobin (HbAlc) is formed by the combination of hemoglobin (Hb) with blood glucose in red blood cells of human blood. The process of blood glucose combining with hemoglobin to form glycated hemoglobin is irreversible. As the average lifespan of red blood cells in blood circulation is 120 days, the content of glycated hemoglobin can reflect the average blood glucose concentration in the past three months. The content of glycated hemoglobin in the blood and the ratio of the content of glycated hemoglobin to that of hemoglobin can reflect long-term blood glucose levels, which have low biological variations, are not affected by blood glucose fluctuations or stress, and do not require fasting or phlebotomy at specific times, and thus have good patient compliance. Based on the above advantages, HbAlc is widely used in clinical practice as an effective monitoring indicator for diabetes screening and diagnosis and for evaluation of long-term blood glucose control efficacy.

Fully automatic biochemical analyzers for detecting glycated hemoglobin have advantages such as fast speed, high throughput, and low costs. Specific methods of the detection include an immunoturbidimetric method and an enzymatic method. The principle of the immunoturbidimetric method is to utilize an agglutination caused by binding of HbAlc to a corresponding monoclonal antibody, which is prepared by using glycosylated amino acids at the N-terminus of the glycated hemoglobin β chain (usually the first 4-10 amino acids) as an antibody recognition site, and to detect a change in the turbidity of the system to quantitatively determine the HbAlc content. In the enzymatic method, the connection between the N-terminal leucine and histidine of the glycated hemoglobin β chain is cut off with a protease to form a glycated dipeptide fragment, which produces hydrogen peroxide under the action of fructosyl peptide oxidase (FPOX), and then produces a color under the reaction with a color developer with the presence of a peroxidase, and the color is proportional to the HbAlc content, so that an absorbance is detected to obtain the HbAlc content and the percentage thereof relative to the total hemoglobin.

In whichever of the immunoturbidimetric method or the enzymatic method, the concentrations of HbAlc and Hb are measured separately or simultaneously, and then converted into standardized results of IFCC or NGSP through ratio calculation.

Since HbAlc in the blood is the combined product of Hb with blood glucose, the accuracy of the measured HbAlc content is affected by the Hb content, which fluctuates over a large range in the blood. For some subjects, the Hb content may be 60 g/L or lower, or even 30 g/L or lower. The accuracy of the measured HbAlc content in a blood sample with a low Hb content is not high.

According to statistics of a large number of clinical samples, the distribution of Hb contents in clinical test samples fluctuates within a range of 20-230 g/L. At low Hb concentrations, the credibility of test results with whichever the enzymatic method or the immunoturbidimetric method is seriously affected, which in turn leads to clinical misjudgment. FIG. 1 shows that for a commercially available HbAlc detecting reagent, measured concentrations of Hb (vertical axis) and Hb levels in samples (horizontal axis) have a very good fitting linearity, while measured concentrations of HbAlc first gradually decreases as the Hb levels in the samples decrease, but suddenly increase when the Hb levels finally approach zero.

HbAlc detecting reagents currently on the market substantially have a nominal applicable range of an Hb content above 60 g/L. Reagents from a few manufacturers are nominally applicable to an Hb content as low as 60 g/L. There are even reagents from some manufacturers are nominally applicable to detecting HbAlc in samples with an Hb content low to 40 g/L, but the results in clinical practice are not ideal. Referring to FIG. 2, a reagent of a manufacturer is nominally applicable to samples with Hb contents of 40 g/L or more. However, when standard substances are used to simulate different hematocrit (HCT) distributions, as an HCT proportion decreases, a deviation of HbAlc detecting results increases.

Therefore, it is necessary to improve HbAlc detecting reagents for fully automatic biochemical analyzers so that the HbAlc detecting reagents can be applicable to the full range of Hb contents of clinical tests.

## SUMMARY

In view of this, the present disclosure intends to provide a reagent that is applicable to a fully automatic biochemical analyzer to detect glycated hemoglobin in blood samples with a full range of hemoglobin contents. The reagent is suitable for an enzymatic method and an immunoturbidimetric method, and can obtain accurate results at any hemoglobin contents, particularly at low hemoglobin contents. The present disclosure further provides a method for detecting glycated hemoglobin using the reagent and use of the reagent.

In a first aspect, the present disclosure provides a reagent for detecting of glycated hemoglobin by a turbidimetric method, comprising at least one amphiphilic compound selected from compound A and compound B, in which the compound A is a compound represented by the following formula A:

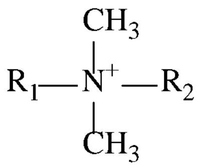

(A)

$R_1$ is selected from a C10-C20 alkyl and $-(CH_2)_{1-3}-N(H)C(O)-R_3$, $R_3$ is selected from a C8-C14 alkyl; and $R_2$ is selected from $-(CH_2)_{1-3}COO^-$ and $-(CH_2)_{1-3}SO_3^-$, and

the compound B is a copolymer of an olefin monomer containing a phosphorylcholine group and an acryl-based or methacryl-based monomer.

According to some embodiments, $R_1$ is selected from a C12-C18 alkyl and $-(CH_2)_3-N(H)C(O)-R_3$, wherein $R_3$ is selected from a C10-C12 alkyl; and $R_2$ is selected from $-(CH_2)_3COO^-$ and $-(CH_2)_3SO_3^-$.

According to some embodiments, the compound B is a copolymer of 2-methacryloyloxyethyl phosphorylcholine and a methacrylate monomer functionalized with a hydrophobic group, an anionic group, or a cationic group.

According to some embodiments, the compound B has a viscosity of 1 to 3 $m^2/s$ and a surface tension of $60\times10^{-3}$ to $70\times10^{-3}$ N/m measured at 25°C with a solution containing 5 wt% of the compound B .

According to some embodiments, a concentration of the compound A in the reagent is 1-10 g/L, preferably 2-8 g/L, and more preferably 5-8 g/L; and/or a concentration of the compound B in the reagent is 1-50 g/L, preferably 15-35 g/L, and more preferably 15-25 g/L.

According to some embodiments, the reagent comprises a hemolytic reagent. Preferably, the reagent comprises: 1-10 g/L of the compound A and 1-50 g/L of the hemolytic reagent. Preferably, the reagent comprises: 1-10 g/L of the compound A, 1-10 g/L of the compound B, and 1-50 g/L of the hemolytic reagent.

According to some embodiments, the hemolytic reagent is at least one quaternary ammonium salt selected from a C8-18 alkyltrimethylammonium halide and optionally the hemolytic reagent further comprises a buffering agent and a preservative. Preferably, a concentration of the quaternary ammonium salt in the hemolytic reagent is 1-50 g/L, preferably 30-50 g/L, and more preferably 40-50 g/L.

According to some embodiments, the reagent further comprises a first turbidimetric HbAlc reagent and a second turbidimetric HbAlc reagent, in which the first turbidimetric HbAlc reagent comprises 0.2-8 mg/ml of an anti-human HbAlc antibody; and the second turbidimetric HbAlc reagent comprises 1-10 μg/mL of an HbAlc polyploid hapten.

According to some embodiments, the reagent further comprises an Hb detecting reagent.

In a second aspect, the present disclosure provides a reagent for detecting glycated hemoglobin in a blood sample, comprising: a turbidimetric sample treatment reagent, which comprises 1-10 g/L of an amphiphilic compound and 1-50 g/L of a hemolytic reagent; a first turbidimetric HbAlc reagent, which comprises 0.2-8 mg/ml of an anti-human HbAlc antibody; and a second turbidimetric HbAlc reagent, which comprises 1-10 μg/mL of an HbAlc polyploid hapten, and in which when the reagent is used to test the blood sample with a hemoglobin content in the range of 300 g/L or less, a test result of glycated hemoglobin has linear consistency.

In some embodiments, the amphiphilic compound is selected from at least one of compound A and compound B, and the compound A and the compound B are as defined in the first aspect above.

In a third aspect, the present disclosure provides a turbidimetric method for detecting glycated hemoglobin, comprising: treating a blood sample using the reagent of the first aspect.

In some embodiments, a hemoglobin content in the blood sample is 300 g/L or less, preferably 20-300 g/L, and more preferably 20-200 g/L.

In some embodiments, the method is a dual detection method or a non-dual detection method.

In some embodiments, the method is a turbidimetric inhibition immunoassay.

In a fourth aspect, the present disclosure provides a reagent for detecting glycated hemoglobin by an enzymatic method, comprising at least one amphiphilic compound selected from compound A' and compound B, in which,

the compound A' is a compound represented by the following formula A':

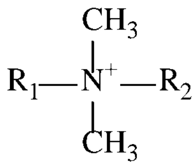

(A')

$R_1$ is selected from a C10-C20 alkyl and $-(CH_2)_{1-3}-N(H)C(O)-R_3$, $R_3$ is selected from a C8-C14 alkyl; and $R_2$ is selected from $-(CH_2)_{1-3}COO^-$; and
the compound B is a copolymer of an olefin monomer containing a phosphorylcholine group and an acryl-based or methacryl-based monomer.

According to some embodiments, in the compound A', $R_1$ is selected from a C12-C18 alkyl and $-(CH_2)_3-N(H)C(O)-R_3$, wherein $R_3$ is selected from a C10-C12 alkyl; and $R_2$ is $-CH_2COO^-$.

According to some embodiments, the compound B is a copolymer of 2-methacryloyloxyethyl phosphorylcholine and a methacrylate monomer functionalized with a hydrophobic group, an anionic group, or a cationic group.

According to some embodiments, the compound B has a viscosity of 1 to 3 $m^2/s$ and a surface tension of 60 to $70\times10^{-3}$ N/m measured at 25°C with a solution containing 5 wt% of the compound B.

According to some embodiments, the reagent comprises a first enzymatic HbAlc reagent, the first enzymatic HbAlc reagent comprising the compound A' at a concentration of 1-10 g/L, preferably 2-8 g/L, and more preferably 5-8 g/L.

According to some embodiments, the reagent comprises a second enzymatic HbAlc reagent, which comprises the compound B at a concentration of 1-50 g/L, preferably 15-35 g/L, and more preferably 15-25 g/L.

According to some embodiments, the reagent comprises a second enzymatic HbAlc reagent, which comprises the compound B at a concentration of 1-50 g/L, preferably 15-35 g/L, and more preferably 15-25 g/L.

According to some embodiments, the first enzymatic HbAlc reagent further comprises 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

According to some embodiments, the second enzymatic HbAlc reagent further comprises 10-200 KU/L of a fructosyl peptide oxidase and 5-100 KU/L of a peroxidase, and optionally a buffer, a preservative, and a protectant.

According to some embodiments, the reagent further comprises a sample treatment solution containing 1-10 mmol/L of sodium nitrite and optionally a preservative.

According to some embodiments, when the reagent for detecting glycated hemoglobin by an enzymatic method comprises the first enzymatic HbAlc reagent (in which the first enzymatic HbAlc reagent comprises the compound A' at a concentration of 1-10 g/L, preferably 2-8 g/L, and more preferably 5-8 g/L), the reagent further comprises a fourth enzymatic HbAlc reagent, which comprises 10-200 KU/L of a fructosyl peptide oxidase and 5-100 KU/L of a peroxidase, and optionally a buffer, a preservative, and a protectant.

According to some embodiments, when the reagent for detecting glycated hemoglobin by an enzymatic method comprises the second enzymatic HbAlc reagent (in which the second enzymatic HbAlc reagent comprises the compound B at a concentration of 1-50 g/L, preferably 15-35 g/L, and more preferably 15-25 g/L), the reagent further comprises a third enzymatic HbAlc reagent, the third enzymatic HbAlc reagent comprising 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

According to some embodiments, the reagent further comprises an Hb test reagent.

In a fifth aspect, the present disclosure provides a reagent for detecting glycated hemoglobin in a blood sample, comprising: an enzymatic sample treatment reagent, which comprises 1-10 mmol/L of sodium nitrite; a first enzymatic HbAlc reagent, which comprises 200-2000 KU/L of an endo-peptide or exo-peptide protease, 0.1-100 mmol/L of a protease activator, 0.01-10 mmol/L of a color developer, and 0.1-5 g/L of an oxidant; and a second enzymatic HbAlc reagent, which comprises 10-200 KU/L of a fructosyl peptide oxidase and 5-100 KU/L of a peroxidase, in which the first enzymatic HbAlc reagent and/or the second enzymatic HbAlc reagent further comprises 1-50 g/L of an amphiphilic compound, and when the reagent is used to test the blood sample with a hemoglobin content in the range of 300 g/L or less, a test result of glycated hemoglobin has linear consistency.

In some embodiments, the amphiphilic compound is selected from at least one of compound A' and compound B, and the compound A' and the compound B are as defined in the fourth aspect above.

In a sixth aspect, the present disclosure provides an enzymatic method for detecting glycated hemoglobin, comprising treating a blood sample using the reagent of the fourth aspect.

In some embodiments, a hemoglobin content in the blood sample is in the range of 300 g/L or less, 20-300 g/L,

and preferably 20-200 g/L.

In some embodiments, the method is a dual detection method or a non-dual detection method.

In a seventh aspect, the present disclosure provides use of compound A in a method for detecting glycated hemoglobin in a blood sample, in which the compound is represented by the following formula A:

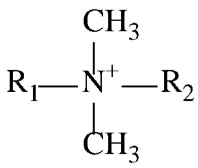

(A)

$R_1$ is selected from a C10-C20 alkyl and $-(CH_2)_{1-3}-N(H)C(O)-R_3$, $R_3$ is selected from a C8-C14 alkyl; and $R_2$ is selected from $-(CH_2)_{1-3}COO^-$ and $-(CH_2)_{1-3}SO_3^-$, and

in which a hemoglobin content in the blood sample is in the range of less than or equal to 60 g/L, preferably less than or equal to 40 g/L, and more preferably less than or equal to 30 g/L.

In the present disclosure, the reagent for detecting HbAlc comprises an amphiphilic compound: a surfactant compound A and/or 2-methacryloyloxyethyl phosphorylcholine copolymer B, and can thus accurately measure the HbAlc content in a sample with a low Hb content, with a test limit as low as an Hb content of 60 g/L or less, or even 30 g/L or less, and can achieve excellent accuracy. The following examples show that the test results of the HbAlc detecting reagent have good linear consistency in an entire range of Hb contents below 300 g/L.

## BRIEF DESCRIPTION OF THE DRAWINGS

In the drawings, the same reference numerals refer to the same or similar components.

FIG. 1 illustrates a linear regression fitting diagram (A) of Hb test results and a linear regression fitting diagram (B) of HbAlc test results of a commercially available HbAlc detecting reagent in clinical tests, in which Hb levels are of a series of samples prepared in reference to the examples.

FIG. 2 is a change curve showing relative deviations of actual test results of a commercially available HbAlc detecting reagent relative to hematocrit percentages (HCT%) in simulated samples.

FIG. 3 illustrates a linear regression fitting diagram (A) of Hb test results and a linear regression fitting diagram (B) of HbAlc test results of Example 2.

FIG. 4 illustrates a linear regression fitting diagram (A) of Hb test results and a linear regression fitting diagram (B) of HbAlc test results of Example 8.

## DETAILED DESCRIPTION

The technical solutions of the present disclosure will be clearly and completely described below in combination with embodiments and examples. Obviously, the specific embodiments described are only some, rather than all, of the embodiments. On the basis of these embodiments, all other embodiments obtained by those skilled in the art without the exercise of any creative labor are within the scope of protection of the present invention.

Throughout the description, unless otherwise specifically indicated, the terms used herein should be understood as having the meanings commonly used in the art. Therefore, unless defined otherwise, all technical and scientific terms used herein have the same meaning as commonly understood by those skilled in the art to which the present invention pertains. In case of conflict, this description shall prevail.

As used herein, the terms "comprise," "contain" or any other variations thereof are intended to encompass a non-exclusive inclusion, such that a method or product comprising a series of elements comprises not only the elements explicitly recited, but also other elements not explicitly listed, or further comprises elements inherent to the practice of the method or product.

As used herein, the singular forms "a", "an" and "the" comprise plural forms of the referenced noun unless otherwise indicated.

The reagent of the present disclosure is accomplished based on the introduction of a zwitterionic compound selected from two categories, one of which is a zwitterionic surfactant containing a quaternary ammonium group, and the other is an acrylic or methacrylic copolymer based on 2-methacryloyloxyethyl phosphorylcholine.

The zwitterionic surfactant is a quaternary ammonium zwitterionic surfactant represented by the following formula A:

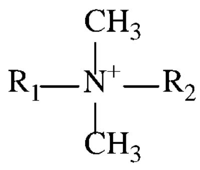

(A)

in which $R_1$ is selected from a C10-C20 alkyl and $-(CH_2)_{1-3}-N(H)C(O)-R_3$, $R_3$ is selected from a C8-C14 alkyl; and $R_2$ is selected from $-(CH_2)_{1-3}COO^-$ and $-(CH_2)_{1-3}SO_3^-$.

More preferably, $R_1$ is selected from a C12-C18 alkyl and $-(CH_2)_3-N(H)C(O)-R_3$, $R_3$ is selected from a C10-C12 alkyl; and $R_2$ is selected from $-CH_2COO^-$ and $-(CH_2)_3SO_3^-$.

Examples of the compound of formula A may be an N-alkyl-N,N-dimethyl-3-ammonium-1-butyrate with the alkyl having 12, 14, 16, or 18 carbon atoms; an N-alkyl-N,N-dimethyl-3-ammonium-1-propionate with the alky having 12, 14, 16, or 18 carbon atoms; lauramidopropyl betaine; an N-alkyl-N,N-dimethyl-3-ammonium-1-propanesulfonate with the alky having 12, 14, 16, or 18 carbon atoms; or a mixture thereof, but are not limited thereto. Commercially available products such as N-(C10-C16 alkyl)-N,N-dimethylglycine betaine, N,N-dimethyl-N-dodecylglycine betaine, etc. can also be used.

A concentration of the compound A in the reagent is 1-10 g/L, preferably 2-8 g/L, and more preferably 5-8 g/L.

The zwitterionic copolymer based on 2-methacryloyloxyethyl phosphorylcholine, i.e., the compound B, is a copolymer of an olefin monomer containing a phosphorylcholine group and an acryl-based or methacryl-based monomer. More specifically, the compound B is a copolymer of 2-methacryloyloxyethyl phosphorylcholine and a methacrylate monomer functionalized with a hydrophobic group, an anionic group, or a cationic group.

The compound B is represented by the following formula B

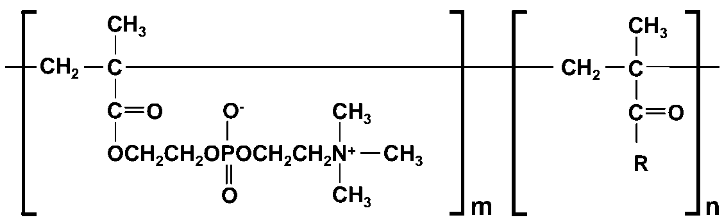

In formula B, -R is the hydrophobic group, anionic group, or cationic group. By adjusting the number of m and n, a series of compounds can be obtained with a specific viscosity (1 to 50 $m^2/s$, measured at 25°C with a solution containing 5 wt% of the compound B) and surface tension ($30\times10^{-3}$ to $80\times10^{-3}$ N/m, measured at 25°C with a solution containing 5 wt% of the compound B).

According to some preferred examples, the compound B used in the present disclosure has a viscosity of 1 to 3 $m^2/s$ and a surface tension of $60\times10^{-3}$ to $70\times10^{-3}$ N/m measured at 25°C with a solution containing 5 wt% of the compound B.

The compound is in particular a product of the Biolipidure® series. Examples of the compound B may be Biolipidure®-1002, Biolipidure®-1301, etc.

A concentration of the compound B in the reagent is 1-50 g/L, preferably 15-35 g/L, and more preferably 15-25 g/L.

According to the embodiments of the present disclosure, the compounds A and B at the above-mentioned concentrations are added alone or in combination to a conventional glycated hemoglobin detecting reagent to obtain a surprisingly accuracy of the measurement and the result is not affected by the Hb content in a sample.

The following describes the embodiments of the present disclosure with respect to an immunoturbidimetric method and an enzymatic method, respectively.

### Turbidimetric method

According to the present disclosure, a reagent for detecting glycated hemoglobin by a turbidimetric method is provided. The reagent comprises at least one zwitterionic compound selected from above-mentioned compound A and compound B.

Glycated hemoglobin reagents for a turbidimetric method typically comprise dual detecting reagents and non-dual detecting reagents, depending on different types or settings of fully automatic biochemical analyzers used.

A dual detecting reagent for a turbidimetric method utilize a set of reagents (kit) to obtain detecting results of the concentrations of Hb and HbAlc and a ratio therebetween simultaneously with single test sample. A non-dual detecting reagent utilize a set of reagents (kit) to obtain only an HbAlc concentration with one test sample, and utilize an additional Hb

detecting reagent to obtain an Hb concentration with another test sample, and then calculates the ratio based on the two concentrations. In either case, a standardized result of IFCC or NGSP can be obtained by calculation according to the following formulas:

$$(1)\ (\text{mmol/mol HbA1c acc. to IFCC}):\ \text{HbA1c (mmol/mol)} = (\text{HbA1c/Hb}) \times 1000;$$

$$(2)\ (\%\ \text{HbA1c acc. to DCCT/NGSP}):\ \text{HbA1c}\ (\%) = (\text{HbA1c/Hb}) \times 91.5 + 2.15.$$

The turbidimetric HbA1c dual detecting reagent and non-dual detecting reagent may be the same, both comprising a sample treatment reagent (i.e., a hemolytic reagent), a first turbidimetric HbAlc reagent, and a second turbidimetric HbAlc reagent. An Hb detecting reagent used in combination with the HbAlc non-dual detecting reagent may be any conventional reagent for detecting Hb content.

The hemolytic reagent can lyse red blood cells and release hemoglobin. The hemolytic reagent is at least one quaternary ammonium salt selected from C8-18 alkyltrimethylammonium halides, in which the halogen is particularly chlorine or bromine. Examples of the lysing agent are dodecyltrimethylammonium chloride, dodecyltrimethylammonium bromide, etc., but are not limited thereto. The hemolytic reagent is present in a red blood cell lysing reagent at a concentration of 1-50 g/L, preferably 30-50 g/L, and more preferably 30-40 g/L.

The hemolytic reagent further comprises a buffering agent such as a phosphate, MES, Tris, HEPES, PIPES, MOPS, Tricine, and TEA. The present disclosure imposes no particular limitation on the specimens of buffering agents. The use amount of the buffering agent is in a range of 0-100 mM. The specific amount varies according to different systems, as long as the pH value of the solution can be maintained within a specific range.

In addition, the hemolytic reagent may further comprise a preservative. Examples of the preservative are sodium azide, ProClin, and sodium benzoate, but not limited thereto. The present disclosure imposes no particular limitation on the specimens of preservatives. The concentration of the preservative in the lysing reagent may be in a range of 0.5-0.95 wt% o.

According to some embodiments, at least one zwitterionic compound selected from the above-mentioned compound A and compound B is added to the red blood cell lysing reagent at the above-mentioned concentration. Preferably, the red blood cell lysing reagent comprises the compound A, or comprises the compound A and the compound B.

The first turbidimetric HbAlc reagent may be any known reagent, and the present disclosure imposes no particular limitation thereon.

For example, the first turbidimetric HbAlc reagent comprises an anti-human HbAlc antibody, such as an anti-human HbAlc antibody (sheep), and the concentration of the antibody is 0.2-8 mg/ml. In addition, the first turbidimetric HbAlc reagent further comprises a buffering agent, a preservative, and a protectant. The buffering agent is, for example, a phosphate, MES, Tris, HEPES, PIPES, MOPS, Tricine, TEA, etc. The present disclosure imposes no particular limitation on the specimens of buffering agents. The amount of the buffering agent is in a range of 0.01-100 mM. The specific amount varies according to different systems, as long as the pH value of the solution can be maintained within a specific range. The preservative is selected from sodium azide, ProClin, sodium benzoate, etc., but is not limited thereto. The present disclosure imposes no particular limitation on the specimens of preservatives. The concentration of the preservative in the first turbidimetric HbAlc reagent is in a range of 0.5-0.95 wt%o. The protectant is selected from glycerol or, dextran, trehalose, polyethylene glycol, amino acid, etc., but is not limited thereto. The present disclosure imposes no particular limitation on the specimens of protectants. The concentration of the protectant in the first turbidimetric HbAlc reagent is in a range of 1-50 g/L.

The second turbidimetric HbAlc reagent may be any known reagent , and the present disclosure imposes no particular limitation thereon.

For example, the second turbidimetric HbAlc reagent comprises an HbAlc polyploid hapten, the concentration of which is 1-10 μg/mL. The second turbidimetric HbAlc reagent also further comprises a buffering agent, a preservative, and a protectant. The buffering agent is, for example, a phosphate, MES, Tris, HEPES, PIPES, MOPS, Tricine, and TEA. The present disclosure imposes no particular limitation on the specimens of buffering agents. The amount of the buffering agent is in a range of 0.01-100 mM. The specific amount varies according to different systems, as long as the pH value of the solution can be maintained within a specific range. The preservative is selected from sodium azide, ProClin, sodium benzoate, etc., but is not limited thereto. The present disclosure imposes no particular limitation on the specimens of preservatives. The concentration of the preservative in the second turbidimetric HbAlc reagent is in a range of 0.5-0.95 wt%o. The protectant is glycerol or, dextran, trehalose, polyethylene glycol, amino acid, etc., but is not limited thereto. The present disclosure imposes no particular limitation on the specimens of protectants. The concentration of the protectant in the second turbidimetric HbAlc reagent is in a range of 1-50 g/L. In addition, the second turbidimetric HbAlc reagent further comprises 100-150 mM of a salt for forming salt ions to maintain stability of the antibody. Usable salts comprise NaCl or

KCl, etc., but are not limited thereto.

According to one embodiment, the above-mentioned set of reagents (kit) comprising the red blood cell lysing reagent, the first turbidimetric HbAlc reagent, and the second turbidimetric HbAlc reagent is used as an HbAlc dual test reagent (kit). According to another embodiment, the above-mentioned set of reagents (kit) comprising the red blood cell lysing reagent, the first turbidimetric HbAlc reagent, and the second turbidimetric HbAlc reagent is used as an HbAlc non-dual test reagent (kit).

For the latter, in a non-dual detecting situation, the present disclosure further provides a kit for measuring an HbAlc concentration and ratio. The kit further comprises an Hb detecting reagent. The Hb detecting reagent may be any reagent used to measure an Hb concentration in a sample, and the present disclosure imposes no particular limitation thereon. For example, the Hb detecting reagent may contain 0-100 mMol/L of a buffering agent selected from a phosphate, MES, Tris, HEPES, PIPES, MOPS, Tricine, TEA, etc., and 0.5-0.95 wt‰ of a preservative selected from sodium azide, ProClin, sodium benzoate, etc.

Some embodiments of the present disclosure provide an immunoturbidimetric method for detecting glycated hemoglobin using the foregoing reagents (kit), which comprises a step of treating a blood sample with the foregoing reagent containing any one of the compound A and the compound B.

According to the present disclosure, after treating the blood sample with the reagent comprising the compound A and/or the compound B, as demonstrated in the following examples, the detecting glycated hemoglobin with the turbidimetric method can maintain accuracy even when the sample contains hemoglobin as low as 60 g/L. In fact, after the sample is treated with the reagent, the linearity of glycated hemoglobin test results is not substantially affected by the hemoglobin content. Therefore, the turbidimetric method is applicable to any blood sample, in which the hemoglobin content is in a range of 300 g/L or less, preferably 20-300 g/L, and more preferably 20-200 g/L.

Specific steps of the turbidimetric method for detecting glycated hemoglobin are the same as those of known methods. In other words, using the foregoing reagents or kit for the detecting of HbAlc, it is not necessary to introduce any additional steps or change the original detecting conditions.

Depending on different types or settings of fully automatic biochemical analyzers used, the detection method may be a dual detection method or a non-dual detection method.

According to a preferred embodiment, the turbidimetric method is a turbidimetric inhibition immunoassay.

In the turbidimetric method for detecting glycated hemoglobin according to the present disclosure, a whole blood sample can be used. A sample of separated red blood cells can also be used.

Specific detecting steps are well known to those skilled in the art and will not be repeated herein.

**Enzymatic method**

Similar to the turbidimetric method, glycated hemoglobin reagents for an enzymatic method typically comprise dual detecting reagents and non-dual detecting reagents, depending on different types or settings of fully automatic biochemical analyzers used.

A dual detecting reagent of the enzymatic method utilizes a set of reagents (kit) to obtain test results of the concentrations of Hb and HbA1c and the ratio therebetween simultaneously with a single test sample. A non-dual detecting reagent utilizes a set of reagents (kit) to obtain only an HbAlc concentration with one test sample, and utilizes an additional Hb detecting reagent to obtain an Hb concentration with another test sample, and then calculates the ratio based on the two concentrations. In either case, standardized results of IFCC or NGSP can be obtained by calculation according to the above formula (1) or (2).

According to the present disclosure, a reagent for detecting glycated hemoglobin by an enzymatic method is provided. The reagent comprises at least one zwitterionic compound selected from compound A' and compound B. Preferably, the reagent comprises the compound A', and more preferably, the reagent comprises the compound A' and the compound B.

The compound A' suitable for detecting of HbAlc by the enzymatic method also has the structure of the above formula A, with only substituents being different:

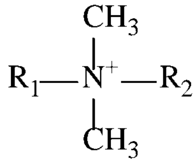

(A')

in which $R_1$ is selected from a C10-C20 alkyl and $-(CH_2)_{1-3}-N(H)C(O)-R_3$, $R_3$ is selected from a C8-C14 alkyl; and $R_2$ is selected from $-(CH_2)_{1-3}COO^-$.

More preferably, $R_1$ is selected from a C12-C18 alkyl and $-(CH_2)_3$-N(H)C(O)-$R_3$, $R_3$ is selected from a C10-C12 alkyl; and $R_2$ is $-CH_2COO^-$.

Examples of the compound A' are an N-alkyl-N,N-dimethyl-3-ammonium-1-butyrate with the alkyl having 12, 14, 16, or 18 carbon atoms; an N-alkyl-N,N-dimethyl-3-ammonium-1-propionate with the alkyl having 12, 14, 16, or 18 carbon atoms; lauramidopropyl betaine; or a mixture thereof, but are not limited thereto. Commercially available products such as N-(C10-C16 alkyl)-N,N-dimethylglycine betaine, N,N-dimethyl-N-dodecylglycine betaine, etc. can also be used.

The concentration of the compound A' in the reagent for detecting HbAlc by an enzymatic method is also 1-10 g/L, preferably 2-8 g/L, and more preferably 5-8 g/L.

The compound B is the same as the above and will not be repeated herein.

The enzymatic HbAlc dual detecting reagent and non-dual detecting reagent may be the same, both comprising a sample treatment solution, a first enzymatic HbAlc reagent, and a second enzymatic HbAlc reagent. An Hb reagent used in combination with the HbAlc non-dual detecting reagents may be any conventional reagent for detecting Hb content.

The sample treatment solution in the reagent for the enzymatic method may be any sample treatment solution commonly used in enzymatic HbAlc detecting reagents, and the present disclosure imposes no particular limitation thereon. Exemplarily, the sample treatment solution comprises 1-10 mM, preferably 3-5 mM, of nitrite; 0-20 nM of a buffering agent and 0.5-0.95‰ of a preservative.

The first enzymatic HbAlc reagent comprises 200-2000 KU/L of an endo-peptide or exo-peptide protease, 0.1-100 mmol/L of a protease activator, 0.01-10 mmol/L of a color developer, and 0.1-5 g/L of an oxidant. The endo-peptide or exo-peptide protease may be, for example, proteinase K, pronase, thermolysin, subtilisin, and bovine trypsin, but is not limited thereto. The protease activator may be, for example, calcium chloride. The color developer may be, for example, N-(carboxymethylaminocarbonyl)-4,4-bis(dimethylamino)-diphenylamine sodium salt (DA-64 for short) and 10-(carboxymethylaminocarbonyl)-3,7-bis(dimethylamino)phenothiazine sodium salt (DA-67 for short), but is not limited thereto. The oxidant may be, for example, maleimide, but is not limited thereto. The present disclosure imposes no particular limitation on the specimens of these reagents, and any reagents conventionally used in the art may be used for the first enzymatic HbAlc reagent of the present disclosure.

The first enzymatic HbAlc reagent may further comprise a buffering agent, a preservative, and a protectant. The buffering agent is, for example, a phosphate, MES, Tris, HEPES, PIPES, MOPS, Tricine, and TEA. The present disclosure imposes no particular limitation on the specimens of buffering agent. The amount of the buffering agent used may be in a range of 0-100 mM. The specific amount varies according to different systems, as long as the pH value of the solution can be maintained within a specific range. The preservative is selected from sodium azide, ProClin, sodium benzoate, etc., but is not limited thereto. The present disclosure imposes no particular limitation on the specimens of preservatives. The concentration of the preservative in the first enzymatic HbAlc reagent mis in a range of 0.5-0.95 wt%o. The protectant is selected from glycerol or, dextran, trehalose, polyethylene glycol, amino acid, etc., but is not limited thereto. The present disclosure imposes no particular limitation on the specimens of protectants. The concentration of the protectant in the first enzymatic HbAlc reagent is in a range of 1-50 g/L.

According to one embodiment of the present disclosure, the first enzymatic HbAlc reagent comprises at least one of the above-mentioned compound A' at the above-mentioned concentration. According to this embodiment, when a blood sample is treated with the first enzymatic HbAlc reagent comprising the compound A', accurate HbAlc measurement result can be obtained at any Hb level.

The second enzymatic HbAlc reagent comprises 10-200 KU/L of a fructosyl peptide oxidase and 5-100 KU/L of a peroxidase. The second enzymatic HbAlc reagent may also further comprise a buffering agent, a preservative, and a protectant. The buffering agent, the preservative, and the protectant used in the second enzymatic HbAlc reagent are the same as those used in the first enzymatic HbAlc reagent, and are not particularly limited in the present disclosure, and are therefore not repeated herein.

According to one embodiment of the present disclosure, the second enzymatic HbAlc reagent comprises at least one of the above-mentioned compound B at the above-mentioned concentration. According to this embodiment, the first enzymatic HbAlc reagent also comprises the compound A'. When the blood sample is treated with the first enzymatic HbAlc reagent comprising the compound A' and the second enzymatic HbAlc reagent comprising the compound B, accurate detection result of HbAlc can be obtained at any Hb level. In this embodiment, the compound A' and the compound B are used at the same time, and the accuracy of the detection result of HbAlc is higher.

According to other embodiments, the first enzymatic HbA1c reagent does not comprise the compound A'. In this embodiment, since the second enzymatic HbAlc reagent comprises at least one compound B, accurate detection result of HbAlc can also be obtained at any Hb level.

According to one embodiment, the above-mentioned set of reagents (kit) comprising the red blood cell lysing reagent, the first enzymatic HbAlc reagent, and the second enzymatic HbAlc reagent is used as an HbAlc dual test reagents (kit). According to another embodiment, the above-mentioned set of reagents (kit) comprising the red blood cell lysing reagent, the first enzymatic HbAlc reagent, and the second enzymatic HbAlc reagent is used as an HbAlc non-dual test reagents (kit).

For the latter, the present disclosure further provides a kit for measuring the HbAlc concentration and the ratio in the non-dual detecting situation. The kit further comprises an Hb detecting reagent. The Hb detecting reagent may be any reagent used to measure an Hb concentration in a sample, and the present disclosure imposes no particular limitation thereon. For example, the Hb detecting reagent may be similar to that used in the above-mentioned turbidimetric method, containing 0-100 mMol/L of a buffering agent selected from a phosphate, MES, Tris, HEPES, PIPES, MOPS, Tricine, TEA, etc., and 0.5-0.95 wt%o of a preservative selected from sodium azide, ProClin, sodium benzoate, etc.

Some embodiments of the present disclosure provide a enzymatic method for detecting glycated hemoglobin using the foregoing reagents (kit), which comprises treating a blood sample with the foregoing reagents containing any one of the compound A' and the compound B.

According to the present disclosure, after treating the blood sample with the reagent comprising the compound A' and/or the compound B, as demonstrated in the following examples, the enzymatic method for detecting glycated hemoglobin can maintain accuracy even when the sample contains hemoglobin at a content of 60 g/L or lower. In fact, after the sample is treated with the reagent, the linearity of glycated hemoglobin detection result is not substantially affected by the hemoglobin content. Therefore, the enzymatic method is applicable to any blood sample, in which the hemoglobin content is in a range of 300 g/L or less, preferably 20-300 g/L, and more preferably 20-200 g/L.

Specific steps of the enzymatic method for detecting glycated hemoglobin are the same as those of known methods. In other words, using the foregoing set of reagents or kit for detecting HbAlc, it is not necessary to introduce additional steps or change the original detecting conditions.

Depending on different types or settings of fully automatic biochemical analyzers used, the detection method may be a dual detection method or a non-dual detection method.

In the enzymatic method for detecting glycated hemoglobin according to the present disclosure, a whole blood sample may be used. A sample of separated red blood cells may also be used.

Specific detecting steps are well known to those skilled in the art and will not be repeated herein.

The embodiments of the present disclosure further provide use of the compound represented by the above formula A in a method for detecting glycated hemoglobin in a blood sample.

According to the following embodiments, when the reagent for detecting glycated hemoglobin, being whichever of a reagent for the turbidimetric method or a reagent for the enzymatic method, contains at least one compound represented by formula A at the above-mentioned concentration, accurate glycated hemoglobin detection result can be obtained even when a hemoglobin content in a sample is at a low level. The hemoglobin content in the sample being at a low level means that the hemoglobin content in the blood sample is in a range of less than or equal to 60 g/L, preferably less than or equal to 40 g/L, and more preferably less than or equal to 30 g/L.

Preferably, the reagent for detecting glycated hemoglobin, being whichever of a reagent for the turbidimetric method or a reagent for the enzymatic method, further comprises at least one compound B at the above-mentioned concentration.

The following is a detailed description of various aspects of the present disclosure through specific embodiments. The features and advantages of various aspects of the present disclosure will become clearer and more apparent through the description of the following embodiments.

**Example 1 Enzymatic dual detecting reagent comprising compound A**

Each reagent was prepared according to the following formulations.

Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Compound A | 1-10 g/L |
| Sodium azide | 0.09% |

The compound A is N,N-dimethyl-N-dodecylglycine betaine.
Second enzymatic HbAlc reagent:

| | |
|---|---|
| Tris buffer (pH 6.5) | 25 mmol/L |
| Fructosyl peptide oxidase | 20 KU/L |
| Peroxidase | 10 KU/L |
| Proclin300 | 0.09% |
| Note: Three set of reagents with the compound A at concentrations of 1 g/L (1-1), 10 g/L (1-2), and 6 g/L (1-3) were prepared, respectively. | |

**Example 2 Enzymatic dual detecting reagent comprising compounds A and B**

Each reagent was prepared according to the following formulations.
Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Compound A | 1-10 g/L |
| Sodium azide | 0.09% |

The compound A is N,N-dimethyl-N-dodecylglycine betaine.
Second enzymatic HbAlc reagent:

| | |
|---|---|
| Tris buffer (pH 6.5) | 25 mmol/L |
| Fructosyl peptide oxidase | 20 KU/L |
| Compound B | 1-50 g/L |
| Peroxidase | 10 KU/L |
| Proclin300 | 0.09% |

The compound B is Biolipidure®-1301.
Note: Three set of reagents with the compound A at a concentration of 6 g/L and the compound B at a concentration of 1 g/L (2-1); the compound A at a concentration of 6 g/L and the compound B at a concentration of 50 g/L (2-2); and the compound A at a concentration of 6 g/L and the compound B at a concentration of 25 g/L (1-3) were prepared, respectively.

**Example 3 Enzymatic dual detecting reagent comprising compound B**

Each reagent was prepared according to the following formulations.
Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Sodium azide | 0.09% |

Second enzymatic HbAlc reagent:

| | |
|---|---|
| Tris buffer (pH 6.5) | 25 mmol/L |
| Fructosyl peptide oxidase | 20 KU/L |
| Compound B | 25 g/L |
| Peroxidase | 10 KU/L |
| Proclin300 | 0.09% |

The compound B is Biolipidure®-1301.

**Example 4 Enzymatic non-dual detecting reagent comprising compound A**

Each reagent was prepared according to the following formulations.

Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Compound A | 6 g/L |
| Sodium azide | 0.09% |

The compound A is N,N-dimethyl-N-dodecylglycine betaine.

Second enzymatic HbAlc reagent:

| | |
|---|---|
| Tris buffer (pH 6.5) | 25 mmol/L |
| Fructosyl peptide oxidase | 20 KU/L |
| Peroxidase | 10 KU/L |
| Proclin300 | 0.09% |

Hb reagent:

| | |
|---|---|
| Phosphate buffer (pH 7.4) | 0.02 mol/L |
| Proclin300 | 0.09% |

**Example 5 Enzymatic non-dual detecting reagent comprising compound B**

Each reagent was prepared according to the following formulation.

Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Sodium azide | 0.09% |

Second enzymatic HbAlc reagent:

| | |
|---|---|
| Tris buffer (pH 6.5) | 25 mmol/L |
| Fructosyl peptide oxidase | 20 KU/L |
| Compound B | 25 g/L |
| Peroxidase | 10 KU/L |
| Proclin300 | 0.09% |

The compound B is Biolipidure®-1301.

Hb reagent:

| | |
|---|---|
| Phosphate buffer (pH 7.4) | 0.02 mol/L |
| Proclin300 | 0.09% |

**Comparative Example 1 Conventional enzymatic dual detecting reagent**

Each reagent was prepared according to the following formulations.

Sample treatment solution:

| | |
|---|---|
| Sodium nitrite | 5 mmol/L |
| Preservative | 0.1 g/L |

First enzymatic HbAlc reagent:

| | |
|---|---|
| Tris buffer (pH 6.5) | 50 mmol/L |
| Proteinase K | 1 MU/L |
| Calcium chloride | 5 mmol/L |
| DA67 | 1 mmol/L |
| Sodium azide | 0.09% |

Second enzymatic HbAlc reagent:

| | |
|---|---|
| Tris buffer (pH 6.5) | 25 mmol/L |
| Fructosyl peptide oxidase | 20 KU/L |
| Peroxidase | 10 KU/L |
| Proclin300 | 0.09% |

**Test Example 1 Detecting Hb and HbA1c in samples with enzymatic method**

(1) dual detecting reagent:

A test was performed on Mindray BS-800 fully automatic biochemical analyzer, specifically comprising the following steps.

200 μL of a sample treatment solution was added to a first reaction vessel, and 10 μL of a whole blood sample was suctioned (a calibrator was used as a sample in a calibration tube), added, and mixed evenly, followed by incubation at 37°C for 5 min to prepare a hemolysate.

A first enzymatic HbAlc reagent was added to a second reaction vessel. An absorbance A1 was read at 505 nm. 12 μL of the hemolysate was suctioned and mixed evenly with 180 μL of the first enzymatic HbAlc reagent, followed by incubation at 37°C for 5 min. An absorbance A2 was read at 505 nm, and an absorbance A3 at 660 nm was acquired at the same time. 60 μL of a second enzymatic HbAlc reagent was then added and mixed well, followed by reaction at 37°C for 5 min, and an absorbance A4 was read at 660 nm.

An Hb concentration was calculated according to the following formula:

Hb concentration = measured absorbance (A2-A1) × [calibration solution] / calibrated absorbance (A2-A1).

Further, an HbAlc concentration was calculated according to the following formula:

HbAlc concentration = measured absorbance (A4-A3) × [calibration solution] / calibrated absorbance (A4-A3).

Finally, an HbA1C percentage concentration was obtained by conversion using an HbA1c/Hb ratio according to the above-mentioned NGSP formula (Formula (2)).

(2) Non-dual detecting reagent:

A test was performed on Mindray BS-800 fully automatic biochemical analyzer, specifically comprising the following steps.

200 μL of a sample treatment solution was added to a first reaction vessel, and 10 μL of a whole blood sample was suctioned (a calibrator was used as a sample in a calibration tube), added, and mixed evenly, followed by incubation at 37°C for 5-10 min to prepare a hemolysate.

180 μL of the first enzymatic HbAlc reagent was added to a second reaction vessel, and 12 μL of the hemolysate was suctioned and mixed evenly, followed by incubation at 37°C for 5 min. An absorbance A1 was read at 660 nm. 60 μL of the second enzymatic HbAlc reagent was then added and mixed well, followed by reaction at 37°C for 5 min. An absorbance A2 was read at 660 nm.

An HbAlc concentration was calculated according to the following formula:

HbAlc concentration = measured absorbance (A2-A1) × [calibration solution] / calibrated absorbance (A2-A1).

180 μL of the Hb detecting reagent was added to a third reaction vessel, and an absorbance A3 was read at 505 nm. 12 μL of the hemolysate was suctioned and mixed evenly, followed by incubation at 37°C for 5 min, and an absorbance A4 was read at 505 nm.

An Hb concentration was calculated according to the following formula:

Hb concentration = measured absorbance (A4-A3) × [calibration solution] / calibrated absorbance (A4-A3).

An HbA1C percentage concentration was obtained by conversion using an HbA1c/Hb ratio according to the above-mentioned NGSP formula (Formula (2)).

The tested samples were a series of samples with different Hb levels obtained by dilution, and were tested on a fully automatic biochemical analyzer using the reagents of the above Comparative Example and Examples 1 to 5 respectively to obtain the Hb concentration and HbAlc concentration. Moreover, a linear regression value and a linear regression relative deviation were calculated. The test results are shown in Tables 1 to 4 below. The linear regression fitting results of Example 2 are shown in FIG. 3. Samples with different Hb levels were prepared with initial sample A (a normal saline with an Hb concentration of 0) and initial sample B (with an Hb concentration of 215 mol/L) according to the dilution ratios shown in Table 1 below. For example, in the following tables, a sample level of 1 and a sample dilution ratio of 7A:1B means a sample obtained by mixing solution A and solution B in a volume ratio of 7:1.

Table 1. Test results with the reagent in Comparative Example 1

| **Sample gradient dilution** | | **Comparative Example 1 Hb concentration (mol/L)** | | | **Comparative Example 1 HbA1c concentration (mmol/L)** | | |
|---|---|---|---|---|---|---|---|
| Sample level | Sample dilution ratio | Measured value | Linear regression value | Linear regression relative deviation | Measured value | Linear regression value | Linear regression relative deviation |
| 0 | 8A:0B | 4.0 | 7.7 | / | 2.5 | 1.4 | / |
| 0.5 | 7.5A:0.5B | 20.7 | 20.7 | 0.0% | 2.0 | 1.6 | 21.2% |
| 1 | 7A:1B | 34.9 | 33.8 | 3.3% | 1.7 | 1.9 | -12.1% |
| 1.5 | 6.5A:1.5B | 48.3 | 46.9 | 3.0% | 1.6 | 2.2 | -26.2% |
| 2 | 6A:2B | 61.3 | 59.9 | 2.2% | 1.9 | 2.5 | -22.9% |
| 3 | 5A:3B | 86.3 | 86.1 | 0.2% | 2.6 | 3.0 | -13.3% |
| 4 | 4A:4B | 113.1 | 112.2 | 0.8% | 3.4 | 3.6 | -6.1% |
| 5 | 3A:5B | 139.0 | 138.4 | 0.5% | 4.1 | 4.1 | -1.2% |
| 6 | 2A:6B | 163.4 | 164.5 | -0.7% | 4.7 | 4.7 | 0.7% |
| 7 | 1A:7B | 190.8 | 190.6 | 0.1% | 5.5 | 5.3 | 4.9% |
| 8 | 0A:8B | 215.9 | 216.8 | -0.4% | 6.1 | 5.8 | 5.1% |

Table 2. Test results with the reagents in Example 1

| Sample gradient dilution | Example 1 (1-1) | | | Example 1 (1-2) | | | Example 1 (1-3) | | |
|---|---|---|---|---|---|---|---|---|---|
| | HbA1c concentration (mmol/L) | | | HbA1c concentration (mmol/L) | | | HbA1c concentration (mmol/L) | | |
| Sample level | Measured value | Linear regression value | Linear regression relative deviation | Measured value | Linear regression value | Linear regression relative deviation | Measured value | Linear regression value | Linear regression relative deviation |
| 0 | 0.5 | 0.1 | / | -0.6 | 0.0 | / | 0.2 | 0.0 | / |
| 0.5 | 0.2 | 0.5 | -57.8% | 0.5 | 0.4 | 18.4% | 0.4 | 0.5 | -8.8% |
| 1 | 0.9 | 0.9 | -4.2% | 0.9 | 0.8 | 11.8% | 0.8 | 0.9 | -6.6% |
| 1.5 | 1.2 | 1.3 | -7.8% | 1.3 | 1.2 | 8.0% | 1.2 | 1.3 | -9.2% |
| 2 | 1.6 | 1.7 | -6.9% | 1.8 | 1.6 | 7.0% | 1.6 | 1.7 | -4.8% |
| 3 | 2.6 | 2.6 | 0.0% | 2.7 | 2.4 | 9.6% | 2.6 | 2.5 | 1.1% |
| 4 | 3.3 | 3.4 | -1.6% | 3.5 | 3.2 | 6.5% | 3.3 | 3.4 | -1.1% |
| 5 | 4.3 | 4.2 | 2.8% | 4.1 | 4.0 | 2.2% | 4.3 | 4.2 | 2.9% |
| 6 | 5.1 | 5.0 | 1.8% | 4.8 | 4.8 | -0.4% | 5.1 | 5.0 | 1.7% |
| 7 | 5.9 | 5.9 | 1.4% | 5.6 | 5.6 | -1.1% | 5.9 | 5.9 | 1.1% |
| 8 | 6.6 | 6.7 | -1.7% | 6.2 | 6.4 | -3.8% | 6.6 | 6.7 | -2.1% |

Table 3. Test results with the reagents in Comparative Example and Example 2

| Sample gradient dilution | Example 2 (2-1) | | | Example 2 (2-2) | | | Example 2 (2-3) | | |
|---|---|---|---|---|---|---|---|---|---|
| | HbA1c concentration (mmol/L) | | | HbA1c concentration (mmol/L) | | | HbA1c concentration (mmol/L) | | |
| Sample level | Measured value | Linear regression value | Linear regression relative deviation | Measured value | Linear regression value | Linear regression relative deviation | Measured value | Linear regression value | Linear regression relative deviation |
| 0 | 0.2 | 0.1 | / | 0.0 | 0.0 | / | 0.0 | 0.0 | / |
| 0.5 | 0.5 | 0.5 | -9.5% | 0.5 | 0.5 | 8.5% | 0.4 | 0.4 | 1.9% |
| 1 | 0.9 | 0.9 | -6.4% | 0.9 | 0.9 | 3.5% | 0.9 | 0.9 | 2.8% |
| 1.5 | 1.3 | 1.4 | -1.8% | 1.3 | 1.3 | 0.7% | 1.3 | 1.3 | 0.0% |
| 2 | 1.8 | 1.8 | 0.5% | 1.8 | 1.8 | 0.0% | 1.7 | 1.7 | 0.4% |
| 3 | 2.6 | 2.6 | -1.9% | 2.6 | 2.6 | -1.7% | 2.6 | 2.6 | -1.6% |
| 4 | 3.4 | 3.5 | -0.6% | 3.5 | 3.5 | -0.3% | 3.5 | 3.5 | -0.6% |
| 5 | 4.3 | 4.3 | 0.8% | 4.3 | 4.3 | 0.3% | 4.3 | 4.3 | -0.2% |
| 6 | 5.1 | 5.1 | -0.2% | 5.1 | 5.2 | -1.0% | 5.2 | 5.2 | -0.7% |
| 7 | 6.0 | 6.0 | -0.3% | 6.0 | 6.0 | -0.3% | 6.1 | 6.1 | 0.3% |
| 8 | 6.9 | 6.8 | 0.7% | 6.9 | 6.9 | 0.8% | 7.0 | 6.9 | 0.5% |

Table 4. Test results with the reagents in Examples 3 to 5

| Sample gradient dilution | Example 3 | | | Example 4 | | | Example 5 | | |
|---|---|---|---|---|---|---|---|---|---|
| | HbA1c concentration (mmol/L) | | | HbA1c concentration (mmol/L) | | | HbA1c concentration (mmol/L) | | |
| Sample level | Measured value | Linear regression value | Linear regression relative deviation | Measured value | Linear regression value | Linear regression relative deviation | Measured value | Linear regression value | Linear regression relative deviation |
| 0 | -0.16 | 0.11 | / | 0.19 | 0.04 | / | -0.10 | 0.11 | / |
| 0.5 | 0.56 | 0.51 | 8.78% | 0.41 | 0.45 | -9.72% | 0.56 | 0.51 | 9.89% |
| 1 | 0.98 | 0.91 | 7.07% | 0.81 | 0.87 | -6.91% | 0.97 | 0.91 | 5.95% |
| 1.5 | 1.41 | 1.31 | 7.43% | 1.19 | 1.29 | -7.32% | 1.40 | 1.31 | 6.58% |
| 2 | 1.74 | 1.71 | 2.15% | 1.66 | 1.70 | -2.45% | 1.76 | 1.71 | 2.61% |
| 3 | 2.57 | 2.50 | 2.71% | 2.51 | 2.53 | -0.78% | 2.54 | 2.51 | 0.89% |
| 4 | 3.41 | 3.30 | 3.25% | 3.35 | 3.36 | -0.25% | 3.39 | 3.31 | 2.23% |
| 5 | 4.05 | 4.10 | -1.28% | 4.30 | 4.19 | 2.44% | 4.10 | 4.11 | -0.27% |
| 6 | 4.82 | 4.89 | -1.54% | 5.11 | 5.03 | 1.74% | 4.85 | 4.91 | -1.25% |
| 7 | 5.77 | 5.69 | 1.33% | 5.90 | 5.86 | 0.80% | 5.60 | 5.71 | -2.05% |
| 8 | 6.39 | 6.49 | -1.46% | 6.57 | 6.69 | -1.80% | 6.59 | 6.51 | 1.12% |

**Example 6 Turbidimetric dual detecting reagent comprising compound A**

Each reagent was prepared according to the following formulations.

Sample treatment solution:

| | |
|---|---|
| Tetradecyltrimethylammonium bromide | 0.9% |
| Compound A | 10 g/L |

The compound A is N,N-dimethyl-N-dodecylglycine betaine.

First turbidimetric HbAlc reagent:

| | |
|---|---|
| Anti-human HbAlc antibody (sheep) | 0.5 mg/mL |
| MES buffer | 0.025 mol/L |
| Tris buffer (pH 6.2) | 0.015 mol/L |
| Proclin300 | 0.09% |

Second turbidimetric HbAlc reagent:

| | |
|---|---|
| HbA1c polyploid hapten | 8 μg/mL |
| MES buffer | 0.025 mol/L |
| Tris buffer (pH 6.2) | 0.015 mol/L |
| Proclin300 | 0.09% |

**Example 7 Turbidimetric dual detecting reagent comprising compound B**

Each reagent was prepared according to the following formulations.

Sample treatment solution:

| | |
|---|---|
| Tetradecyltrimethylammonium bromide | 0.9% |
| Compound B | 25 g/L |

The compound B is Biolipidure®-1301.

First turbidimetric HbAlc reagent:

| | |
|---|---|
| Anti-human HbA1c antibody (sheep) | 0.5 mg/mL |
| MES buffer | 0.025 mol/L |
| Tris buffer (pH 6.2) | 0.015 mol/L |
| Proclin300 | 0.09% |

Second turbidimetric HbAlc reagent:

| | |
|---|---|
| HbA1c polyploid hapten | 8 μg/mL |
| MES buffer | 0.025 mol/L |
| Tris buffer (pH 6.2) | 0.015 mol/L |
| Proclin300 | 0.09% |

**Example 8 Turbidimetric non-dual detecting reagent comprising compound A**

Each reagent was prepared according to the following formulations.

Sample treatment solution:

| | |
|---|---|
| Tetradecyltrimethylammonium bromide | 0.9% |
| Compound A | 1-10 g/L |

The compound A is N,N-dimethyl-N-dodecylglycine betaine.

First turbidimetric HbAlc reagent:

| | |
|---|---|
| Anti-human HbA1c antibody (sheep) | 0.5 mg/mL |
| MES buffer | 0.025 mol/L |
| Tris buffer (pH 6.2) | 0.015 mol/L |
| Proclin300 | 0.09% |

Second turbidimetric HbAlc reagent:

| | |
|---|---|
| HbA1c polyploid hapten | 8 μg/mL |
| MES buffer | 0.025 mol/L |
| Tris buffer (pH 6.2) | 0.015 mol/L |
| Proclin300 | 0.09% |

Hb reagent:

| | |
|---|---|
| Phosphate buffer (pH 7.4) | 0.02 mol/L |
| Proclin300 | 0.09% |
| Note: Two set of reagents with the compound A at concentrations of 5 g/L (8-1) and 10 g/L (8-2) were prepared, respectively. | |

**Example 9 Turbidimetric non-dual detecting reagent comprising compound B**

Each reagent was prepared according to the following formulations.

Sample treatment solution:

| | |
|---|---|
| Tetradecyltrimethylammonium bromide | 0.9% |
| Compound B | 1-50 g/L |

The compound B is Biolipidure®-1301.

First turbidimetric HbAlc reagent:

| | |
|---|---|
| Anti-human HbA1c antibody (sheep) | 0.5 mg/mL |
| MES buffer | 0.025 mol/L |
| Tris buffer (pH 6.2) | 0.015 mol/L |
| Proclin300 | 0.09% |

Second turbidimetric HbAlc reagent:

| | |
|---|---|
| HbA1c polyploid hapten | 8 μg/mL |
| MES buffer | 0.025 mol/L |
| Tris buffer (pH 6.2) | 0.015 mol/L |
| Proclin300 | 0.09% |

Hb reagent:

| | |
|---|---|
| Phosphate buffer (pH 7.4) | 0.02 mol/L |
| Proclin300 | 0.09% |
| Note: Two set of reagents with the compound A at concentrations of 25 g/L (9-1) and 50 g/L (9-2) were prepared, respectively. | |

**Comparative Example 2 Conventional turbidimetric non-dual detecting reagent**

Each reagent was prepared according to the following formulations.

Sample treatment solution:

| | |
|---|---|
| Tetradecyltrimethylammonium bromide | 0.9% |

First turbidimetric HbAlc reagent:

| | |
|---|---|
| Anti-human HbA1c antibody (sheep) | 0.5 mg/mL |
| MES buffer | 0.025 mol/L |
| Tris buffer (pH 6.2) | 0.015 mol/L |
| Proclin300 | 0.09% |

Second turbidimetric HbAlc reagent:

| | |
|---|---|
| HbA1c polyploid hapten | 8 μg/mL |
| MES buffer | 0.025 mol/L |
| Tris buffer (pH 6.2) | 0.015 mol/L |
| Proclin300 | 0.09% |

Hb reagent:

| | |
|---|---|
| Phosphate buffer (pH 7.4) | 0.02 mol/L |
| Proclin300 | 0.09% |

**Test Example 2 Detecting Hb and HbA1c in samples with turbidimetric method**

(1) dual detecting reagent:

A test was performed on Mindray BS-800 fully automatic biochemical analyzer, specifically comprising the following steps.

200 μL of a sample treatment solution was added to a first reaction vessel, and 2 μL of a whole blood sample was suctioned (a calibrator was used as a sample in a calibration tube), added, and mixed evenly, followed by incubation at 37°C for 5 min to prepare a hemolysate.

200 μL of the first turbidimetric HbAlc reagent was added to a second reaction vessel. An absorbance A1 was read at 570 nm. 8 μL of the hemolysate was suctioned and mixed evenly, followed by incubation at 37°C for 5 min. An absorbance A2 was read at 570 nm, and an absorbance A3 was read at 340 nm at the same time. 40 μL of the second turbidimetric HbAlc reagent was then added and mixed well, followed by reaction at 37°C for 5 min, and an absorbance A4 was then read at 340 nm.

An Hb concentration was calculated according to the following formula:

Hb concentration = measured absorbance (A2-A1) × [calibration solution] / calibrated absorbance (A2-A1).

Further, an HbAlc concentration was calculated according to the following formula:

HbAlc concentration = measured absorbance (A4-A3) × [calibration solution] / calibrated absorbance (A4-A3).

Finally, an HbA1C percentage content was obtained by conversion using an HbA1c/Hb ratio according to the above-mentioned NGSP formula (Formula (2)).

(2) Non-dual detecting reagent:

A test was performed on Mindray BS-800 fully automatic biochemical analyzer, specifically comprising the following steps.

200 μL of a sample treatment solution was added to a first reaction vessel, and 2 μL of a whole blood sample was suctioned (a calibrator was used as a sample in a calibration tube), added, and mixed evenly, followed by incubation at 37°C for 5 min to prepare a hemolysate.

200 μL of the first turbidimetric HbAlc reagent was added to a second reaction vessel, and 8 μL of the hemolysate was suctioned and mixed evenly, followed by incubation at 37°C for 5 min. An absorbance A1 was read at 340 nm. 40 μL of the second turbidimetric HbAlc reagent was then added and mixed well, followed by reaction at 37°C for 5 min. An absorbance A2 was read at 340 nm.

An HbAlc concentration was calculated according to the following formula:

HbAlc concentration = measured absorbance (A2-A1) × [calibration solution] / calibrated absorbance (A2-A1).

184 μL of the Hb test reagent was added to a third reaction vessel, and 16 μL of the hemolysate was suctioned and mixed evenly, followed by incubation at 37°C for 5 min. An absorbance A was read at 570 nm.

An Hb concentration was calculated according to the following formula:

Hb concentration = measured absorbance A × [calibration solution] / calibrated absorbance A.

An HbA1C percentage content was obtained by conversion using an HbA1c/Hb ratio according to the above-mentioned NGSP formula (Formula (2)).

A series of samples with different Hb levels were prepared using the same method as in Test Example 1, and were tested on a fully automatic biochemical analyzer using the reagents of the above Examples 6 to 9 respectively to obtain the Hb concentration and HbAlc concentration. Moreover, a linear regression value and a linear regression relative deviation were calculated. The test results are shown in Tables 5 to 8 below. The linear regression fitting diagrams of Example 8 are shown in FIG. 4.

Table 5. Test results with the reagent in Comparative Example 2

| **Sample gradient dilution** | | **Comparative Example 2** | | | **Comparative Example 2** | | |
|---|---|---|---|---|---|---|---|
| | | **Hb concentration (mol/L)** | | | **HbA1c concentration (mmol/L)** | | |
| Sample level | Sample dilution ratio | Measured value | Linear regression value | Linear regression relative deviation | Measured value | Linear regression value | Linear regression relative deviation |
| 0 | 8A:0B | 5.1 | 6.3 | / | 2.6 | 1.7 | / |
| 0.5 | 7.5A:0.5B | 20.4 | 19.7 | 3.6% | 1.6 | 2.0 | -22.7% |
| 1 | 7A:1B | 33.9 | 33.1 | 2.5% | 2.0 | 2.3 | -14.5% |
| 1.5 | 6.5A:1.5B | 47.7 | 46.5 | 2.6% | 2.3 | 2.6 | -10.1% |
| 2 | 6A:2B | 60.2 | 59.9 | 0.5% | 2.9 | 2.8 | 1.3% |
| 3 | 5A:3B | 86.2 | 86.7 | -0.6% | 3.4 | 3.4 | 0.7% |
| 4 | 4A:4B | 113.2 | 113.5 | -0.3% | 3.9 | 3.9 | -1.2% |
| 5 | 3A:5B | 139.1 | 140.3 | -0.9% | 4.5 | 4.5 | 0.5% |
| 6 | 2A:6B | 165.7 | 167.1 | -0.8% | 5.0 | 5.0 | -1.8% |
| 7 | 1A:7B | 192.7 | 193.9 | -0.6% | 5.7 | 5.6 | 1.4% |

(continued)

| Sample gradient dilution | | Comparative Example 2 Hb concentration (mol/L) | | | Comparative Example 2 HbA1c concentration (mmol/L) | | |
|---|---|---|---|---|---|---|---|
| Sample level | Sample dilution ratio | Measured value | Linear regression value | Linear regression relative deviation | Measured value | Linear regression value | Linear regression relative deviation |
| 8 | 0A:8B | 223.5 | 220.7 | 1.3% | 6.3 | 6.1 | 1.7% |

Table 6. Test results with the reagents in Examples 6 to 7

| Sample gradient dilution | Example 6 HbA1c concentration (mmol/L) | | | Example 7 HbA1c concentration (mmol/L) | | |
|---|---|---|---|---|---|---|
| Sample level | Measured value | Linear regression value | Linear regression relative deviation | Measured value | Linear regression value | Linear regression relative deviation |
| 0 | 0.13 | 0.07 | / | 0.10 | 0.16 | / |
| 0.5 | 0.48 | 0.50 | -4.44% | 0.60 | 0.58 | 4.32% |
| 1 | 0.88 | 0.92 | -4.82% | 1.05 | 0.99 | 5.53% |
| 1.5 | 1.33 | 1.35 | -1.27% | 1.44 | 1.41 | 2.00% |
| 2 | 1.79 | 1.77 | 0.65% | 1.89 | 1.83 | 3.08% |
| 3 | 2.60 | 2.62 | -1.01% | 2.56 | 2.66 | -3.81% |
| 4 | 3.48 | 3.48 | 0.06% | 3.44 | 3.50 | -1.86% |
| 5 | 4.40 | 4.33 | 1.73% | 4.33 | 4.34 | -0.17% |
| 6 | 5.20 | 5.18 | 0.49% | 5.20 | 5.17 | 0.58% |
| 7 | 5.96 | 6.03 | -1.22% | 6.10 | 6.01 | 1.58% |
| 8 | 6.89 | 6.88 | 0.21% | 6.78 | 6.84 | -0.85% |

Table 7. Test results with the reagents in Example 8

| Sample gradient dilution | Example 8 (8-1) HbA1c concentration (mmol/L) | | | Example 8 (8-2) HbA1c concentration (mmol/L) | | |
|---|---|---|---|---|---|---|
| Sample level | Measured value | Linear regression value | Linear regression relative deviation | Measured value | Linear regression value | Linear regression relative deviation |
| 0 | 0.1 | 0.2 | / | 0.1 | 0.1 | / |
| 0.5 | 0.6 | 0.6 | -3.1% | 0.5 | 0.5 | -0.3% |
| 1 | 1.0 | 1.0 | -0.9% | 0.9 | 0.9 | -1.7% |
| 1.5 | 1.4 | 1.4 | 0.0% | 1.3 | 1.3 | -2.3% |
| 2 | 1.8 | 1.8 | -1.2% | 1.7 | 1.7 | 0.3% |
| 3 | 2.6 | 2.6 | 0.3% | 2.5 | 2.5 | -0.6% |
| 4 | 3.5 | 3.4 | 2.8% | 3.4 | 3.4 | 0.2% |
| 5 | 4.2 | 4.2 | 1.0% | 4.2 | 4.2 | 1.1% |

(continued)

| Sample gradient dilution | Example 8 (8-1) | | | Example 8 (8-2) | | |
|---|---|---|---|---|---|---|
| | HbA1c concentration (mmol/L) | | | HbA1c concentration (mmol/L) | | |
| Sample level | Measured value | Linear regression value | Linear regression relative deviation | Measured value | Linear regression value | Linear regression relative deviation |
| 6 | 5.0 | 5.0 | 0.6% | 5.1 | 5.0 | 2.2% |
| 7 | 5.7 | 5.8 | -1.1% | 5.8 | 5.8 | 0.8% |
| 8 | 6.6 | 6.6 | -0.5% | 6.5 | 6.6 | -2.1% |

Table 8. Test results with the reagents in Example 9

| Sample gradient dilution | Example 9 (9-1) | | | Example 9 (9-2) | | |
|---|---|---|---|---|---|---|
| | HbA1c concentration (mmol/L) | | | HbA1c concentration (mmol/L) | | |
| Sample level | Measured value | Linear regression value | Linear regression relative deviation | Measured value | Linear regression value | Linear regression relative deviation |
| 0 | 0.1 | 0.2 | / | 0.2 | 0.2 | / |
| 0.5 | 0.6 | 0.6 | 2.8% | 0.6 | 0.6 | 7.2% |
| 1 | 1.0 | 1.0 | 3.5% | 1.0 | 1.0 | 3.2% |
| 1.5 | 1.4 | 1.4 | 0.1% | 1.4 | 1.4 | -2.8% |
| 2 | 1.8 | 1.8 | -0.6% | 1.8 | 1.8 | -0.5% |
| 3 | 2.5 | 2.6 | -1.0% | 2.6 | 2.6 | -1.6% |
| 4 | 3.4 | 3.4 | 0.9% | 3.4 | 3.4 | 0.5% |
| 5 | 4.2 | 4.2 | 0.9% | 4.2 | 4.2 | -0.1% |
| 6 | 5.1 | 5.0 | 1.5% | 5.1 | 5.0 | 1.9% |
| 7 | 5.8 | 5.8 | 0.3% | 5.8 | 5.8 | 0.3% |
| 8 | 6.5 | 6.6 | -1.6% | 6.5 | 6.6 | -1.1% |

In other aspects, the present disclosure provides any one of the following embodiments.

According to a first embodiment, a reagent for detecting glycated hemoglobin by a turbidimetric method is provided, which comprises at least one amphiphilic compound selected from compound A and compound B, and compound A and compound B are as defined in the first aspect above.

According to some embodiments, a concentration of the compound A in the reagent is 1-10 g/L, preferably 2-8 g/L, and more preferably 5-8 g/L.

According to some embodiments, a concentration of the compound B in the reagent is 1-50 g/L, preferably 15-35 g/L, and more preferably 15-25 g/L.

According to some embodiments, the reagent comprises a hemolytic reagent.

According to some specific embodiments, the reagent comprises: 1-10 g/L of the compound A and 1-50 g/L of the hemolytic reagent.

According to some other specific embodiments, the reagent comprises: 1-10 g/L of the compound A, 1-10 g/L of the compound B, and 1-50 g/L of the hemolytic reagent.

According to some specific embodiments, the hemolytic reagent is at least one quaternary ammonium salt selected from a C8-18 alkyltrimethylammonium halide.

A concentration of the quaternary ammonium salt in the hemolytic reagent is 1-50 g/L, preferably 30-50 g/L, and more preferably 40-50 g/L, and optionally the hemolytic reagent further comprises a buffering agent and a preservative.

According to a second embodiment, a kit for detecting glycated hemoglobin by a turbidimetric agent is provided, comprising any one of the foregoing reagents for detecting of glycated hemoglobin by the turbidimetric method.

According to some embodiments, the kit for detecting glycated hemoglobin by a turbidimetric method further comprises a first turbidimetric HbAlc reagent and a second turbidimetric HbAlc reagent, wherein the first turbidimetric HbAlc reagent comprises 0.2-8 mg/ml of an anti-human HbAlc antibody; and the second turbidimetric HbAlc reagent comprises 1-10 μg/mL of an HbAlc polyploid hapten.

The first turbidimetric HbAlc reagent and the second turbidimetric HbAlc reagent respectively further comprise a buffering agent, a preservative, and a protectant, and the second turbidimetric HbAlc reagent further comprises a salt.

According to some embodiments, the kit for detecting glycated hemoglobin by the turbidimetric method further comprises an Hb test reagent.

According to a third embodiment, a reagent for detecting glycated hemoglobin in a blood sample is provided, which comprises:

a turbidimetric sample treatment reagent, comprising 1-10 g/L of an amphiphilic compound and 1-50 g/L of a hemolytic reagent;
a first turbidimetric HbAlc reagent, comprising 0.2-8 mg/ml of an anti-human HbAlc antibody; and
a second turbidimetric HbAlc reagent, comprising 1-10 μg/mL of an HbAlc polyploid hapten,
in which when the reagent is used to test the blood sample with a hemoglobin content in a range of 300 g/L or less, a detection result of glycated hemoglobin has linear consistency.

According to some embodiments, the amphiphilic compound is selected from at least one of compound A and compound B, wherein the compound A and the compound B are as defined above.

According to a fourth embodiment, a turbidimetric method for detecting glycated hemoglobin is provided, which comprises treating a blood sample using any one of the foregoing reagents for detecting glycated hemoglobin by the turbidimetric method.

According to a specific embodiment, a hemoglobin content in the blood sample is in a range of 300 g/L or less, preferably 20-300 g/L, and more preferably 20-200 g/L.

According to some embodiments, the method comprises a dual detection method or a non-dual detection method.

According to some specific embodiments, the method is a turbidimetric inhibition immunoassay.

According to a fifth embodiment, a reagent for detecting glycated hemoglobin by an enzymatic method is provided, which comprises at least one amphiphilic compound selected from compound A' and compound B, and the compound A' and compound B are as defined in the third aspect above.

According to some embodiments, the reagent for detecting glycated hemoglobin by the enzymatic method comprises a first enzymatic HbAlc reagent, comprising the compound A' at a concentration of 1-10 g/L, preferably 2-8 g/L, and more preferably 5-8 g/L.

According to some other embodiments, the reagent for detecting glycated hemoglobin by the enzymatic method comprises a second enzymatic HbAlc reagent, comprising the compound B at a concentration of 1-50 g/L, preferably 15-35 g/L, and more preferably 15-25 g/L.

According to some further embodiments, the reagent for detecting glycated hemoglobin by the enzymatic method comprises a first enzymatic HbAlc reagent, comprising the compound A' at a concentration of 1-10 g/L, preferably 2-8 g/L, and more preferably 5-8 g/L; and a second enzymatic HbAlc reagent, comprising the compound B at a concentration of 1-50 g/L, preferably 15-35 g/L, and more preferably 15-25 g/L.

The first enzymatic HbAlc reagent further comprises 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

The second enzymatic HbAlc reagent further comprises 10-200 KU/L of a fructosyl peptide oxidase and 5-100 KU/L of a peroxidase, and optionally a buffering agent, a preservative, and a protectant.

According to a sixth embodiment, a kit for detecting glycated hemoglobin by an enzymatic method is provided, which comprises any one of the foregoing reagents for detecting glycated hemoglobin by the enzymatic method.

According to some embodiments, the kit for enzymatic detecting of glycated hemoglobin further comprises a sample treatment solution containing 1-10 mmol/L of sodium nitrite and optionally a preservative.

According to some embodiments, when the reagent for detecting glycated hemoglobin by the enzymatic method comprises the first enzymatic HbAlc reagent (the first enzymatic HbAlc reagent comprises the compound A' at a concentration of 1-10 g/L, preferably 2-8 g/L, and more preferably 5-8 g/L), the kit further comprises a fourth enzymatic HbAlc reagent, the fourth enzymatic HbAlc reagent comprising 10-200 KU/L of a fructosyl peptide oxidase and 5-100 KU/L of a peroxidase, and optionally a buffer, a preservative, and a protectant.

According to some other embodiments, when the reagent for detecting glycated hemoglobin by the enzymatic

method comprises the second enzymatic HbAlc reagent (the second enzymatic HbAlc reagent comprises the compound B at a concentration of 1-50 g/L, preferably 15-35 g/L, and more preferably 15-25 g/L), the kit further comprises a third enzymatic HbAlc reagent, the third enzymatic HbAlc reagent comprising 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

According to some embodiments, the kit further comprises an Hb detecting reagent.

According to a seventh embodiment, a reagent for detecting glycated hemoglobin in a blood sample is provided, comprising:

an enzymatic sample treatment reagent comprising 1-10 mmol/L of sodium nitrite;
a first enzymatic HbAlc reagent comprising 200-2000 KU/L of an endo-peptide or exo-peptide protease, 0.1-100 mmol/L of a protease activator, 0.01-10 mmol/L of a color developer, and 0.1-5 g/L of an oxidant; and
a second enzymatic HbA1c reagent comprising 10-200 KU/L of a fructosyl peptide oxidase and 5-100 KU/L of a peroxidase,
in which the first enzymatic HbAlc reagent and/or the second enzymatic HbAlc reagent further comprises 1-50 g/L of an amphiphilic compound,
and when the reagent is used to test the blood sample with a hemoglobin content in a range of 300 g/L or less, a test result of glycated hemoglobin has linear consistency.

According to some embodiments, the amphiphilic compound is selected from at least one of compound A' and compound B, wherein the compound A' and the compound B are as defined above.

According to an eighth embodiment, an enzymatic method for detecting glycated hemoglobin is provided, which comprises treating a blood sample using any one of the foregoing reagents for detecting glycated hemoglobin by the enzymatic method.

According to some embodiments, a hemoglobin content in the blood sample is in the range of 300 g/L or less, 20-300 g/L, and preferably 20-200 g/L.

According to some embodiments, the method is a dual detection method or a non-dual detection method.

According to a ninth embodiment, use of compound A in a method for detecting glycated hemoglobin in a blood sample is provided, in which the compound A is as defined above, and a hemoglobin content in the blood sample is in a range of less than or equal to 60 g/L, preferably less than or equal to 40 g/L, and more preferably less than or equal to 30 g/L.

According to some embodiments, the method in the use is an enzymatic method or a turbidimetric method.

According to some embodiments, in the use, the method further comprises using compound B at the same time in detecting glycated hemoglobin, in which the compound B is a copolymer of an olefin monomer containing a phosphorylcholine group and an acrylic or methacrylic monomer.

According to some specific embodiments, in the use, the method comprises treating the blood sample with a reagent for detecting glycated hemoglobin by an enzymatic method, wherein the reagent for detecting glycated hemoglobin by the enzymatic method comprises:

an enzymatic sample treatment reagent comprising 1-10 mmol/L of sodium nitrite;
a first enzymatic HbAlc reagent comprising 1-50 g/L of the compound A, 200-2000 KU/L of an endo-peptide or exo-peptide protease, 0.1-100 mmol/L of a protease activator, 0.01-10 mmol/L of a color developer, and 0.1-5 g/L of an oxidant; and
a second enzymatic HbAlc reagent comprising 10-200 KU/L of a fructosyl peptide oxidase and 5-100 KU/L of a peroxidase,
in which the second enzymatic HbAlc reagent further comprises 1-10 g/L of compound B, which is a copolymer of an olefin monomer containing a phosphorylcholine group and an acrylic or methacrylic monomer.

According to some other specific embodiments, in the use, the method comprises treating the blood sample with a reagent for detecting glycated hemoglobin by a turbidimetric method, in which the reagent for detecting glycated hemoglobin by a turbidimetric method comprises:

a turbidimetric sample treatment reagent comprising 1-10 g/L of the compound A and 1-50 g/L of a hemolytic reagent;
a first turbidimetric HbAlc reagent comprising 0.2-8 mg/ml of an anti-human HbAlc antibody; and
a second turbidimetric HbAlc reagent comprising 1-10 $\mu$g/mL of an HbAlc polyploid hapten,
in which the turbidimetric sample treatment reagent further comprises 1-10 g/L of compound B, which is a copolymer of an olefin monomer containing a phosphorylcholine group and an acrylic or methacrylic monomer. Described above are only preferred embodiments of the present disclosure, which therefore do not limit the patent scope of the present disclosure. All equivalent structural variations made by using the contents of the description and the drawings of the

present disclosure under the inventive concept of the present disclosure, or direct/indirect applications to other related technical fields shall all be included in the patent scope of protection of the present disclosure.

## Claims

1. A reagent for detecting glycated hemoglobin by a turbidimetric method, comprising at least one amphiphilic compound selected from compound A and compound B, wherein

   the compound A is represented by the following formula A:

   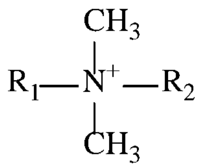

   (A)

   wherein,

   $R_1$ is selected from a C10-C20 alkyl and $-(CH_2)_{1-3}-N(H)C(O)-R_3$, wherein $R_3$ is selected from a C8-C14 alkyl; and
   $R_2$ is selected from $-(CH_2)_{1-3}COO^-$ and $-(CH_2)_{1-3}SO_3^-$, and

   the compound B is a copolymer of an olefin monomer containing a phosphorylcholine group and an acryl-based or methacryl-based monomer.

2. The reagent according to claim 1, wherein $R_1$ is selected from a C12-C18 alkyl and $-(CH_2)_3-N(H)C(O)-R_3$, wherein $R_3$ is selected from a C10-C12 alkyl; and $R_2$ is selected from $-(CH_2)_3COO^-$ and $-(CH_2)_3SO_3^-$.

3. The reagent according to claim 1, wherein the compound B is a copolymer of 2-methacryloyloxyethyl phosphorylcholine and a methacrylate monomer functionalized with a hydrophobic group, an anionic group, or a cationic group.

4. The reagent according to claim 3, wherein the compound B has a viscosity of 1 to 3 $m^2/s$ and a surface tension of $60\times10^{-3}$ to $70\times10^{-3}$ N/m, which are measured at 25°C with a solution containing 5 wt% of the compound B.

5. The reagent according to claim 1, wherein a concentration of the compound A in the reagent is 1-10 g/L, preferably 2-8 g/L, and more preferably 5-8 g/L; and/or a concentration of the compound B in the reagent is 1-50 g/L, preferably 15-35 g/L, and more preferably 15-25 g/L.

6. The reagent according to any one of claims 1 to 5, wherein the reagent comprises a hemolytic reagent;

   preferably, the reagent comprises:
   1-10 g/L of the compound A and 1-50 g/L of the hemolytic reagent; or
   preferably, the reagent comprises:
   1-10 g/L of the compound A, 1-10 g/L of the compound B, and 1-50 g/L of the hemolytic reagent.

7. The reagent according to claim 6, wherein the hemolytic reagent is at least one quaternary ammonium salt selected from a C8-18 alkyltrimethylammonium halide and optionally the hemolytic reagent further comprises a buffering agent and a preservative;
   preferably, a concentration of the quaternary ammonium salt in the hemolytic reagent is 1-50 g/L, preferably 30-50 g/L, and more preferably 40-50 g/L.

8. The reagent according to any one of claims 1 to 7, further comprising a first turbidimetric HbAlc reagent and a second turbidimetric HbAlc reagent, wherein

   the first turbidimetric HbAlc reagent comprises 0.2-8 mg/ml of an anti-human HbAlc antibody; and
   the second turbidimetric HbAlc reagent comprises 1-10 μg/mL of an HbAlc polyploid hapten.

**9.** The reagent according to claim 8, further comprising an Hb detecting reagent.

**10.** A reagent for detecting glycated hemoglobin in a blood sample, comprising:

a turbidimetric sample treatment reagent, which comprises 1-10 g/L of an amphiphilic compound and 1-50 g/L of a hemolytic reagent;
a first turbidimetric HbAlc reagent, which comprises 0.2-8 mg/ml of an anti-human HbAlc antibody; and
a second turbidimetric HbAlc reagent, which comprises 1-10 $\mu$g/mL of an HbAlc polyploid hapten,
wherein when the reagent is used to test the blood sample with a hemoglobin content of 300 g/L or less, and a test result of glycated hemoglobin has linear consistency.

**11.** The reagent according to claim 10, wherein the amphiphilic compound is selected from at least one of compound A and compound B, and the compound A and the compound B are defined according to any one of claims 1 to 4.

**12.** A turbidimetric method for detecting glycated hemoglobin, comprising treating a blood sample using the reagent according to any one of claims 1 to 9.

**13.** The method according to claim 12, wherein a hemoglobin content in the blood sample is 300 g/L or less, preferably 20-300 g/L, and more preferably 20-200 g/L.

**14.** The method according to claim 12 or 13, wherein the method is a dual detection method or a non-dual detection method.

**15.** The method according to any one of claims 12 to 14, wherein the method is a turbidimetric inhibition immunoassay.

**16.** A reagent for detecting glycated hemoglobin by an enzymatic method, comprising at least one amphiphilic compound selected from compound A’ and compound B, wherein

the compound A’ is represented by the following formula A’:

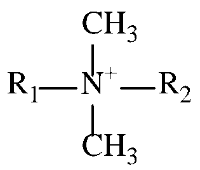

(A')

wherein

$R_1$ is selected from a C10-C20 alkyl and $-(CH_2)_{1-3}-N(H)C(O)-R_3$, wherein $R_3$ is selected from a C8-C14 alkyl; and
$R_2$ is selected from $-(CH_2)_{1-3}COO^-$; and

the compound B is a copolymer of an olefin monomer containing a phosphorylcholine group and an acrylic or methacrylic monomer.

**17.** The reagent according to claim 16, wherein $R_1$ is selected from a C12-C18 alkyl and $-(CH_2)_3-N(H)C(O)-R_3$, wherein $R_3$ is selected from a C10-C12 alkyl; and $R_2$ is $-CH_2COO^-$.

**18.** The reagent according to claim 16, wherein the compound B is a copolymer of 2-methacryloyloxyethyl phosphorylcholine and a methacrylate monomer functionalized with a hydrophobic group, an anionic group, or a cationic group.

**19.** The reagent according to claim 18, wherein the compound B has a viscosity of 1 to 3 $m^2/s$ and a surface tension of $60\times10^{-3}$ to $70\times10^{-3}$ N/m, which are measured at 25°C with a solution containing 5 wt% of the compound B.

**20.** The reagent according to any one of claims 16 to 19, wherein the reagent comprises a first enzymatic HbAlc reagent, which comprises the compound A’ at a concentration of 1-10 g/L, preferably 2-8 g/L, and more preferably 5-8 g/L.

**21.** The reagent according to any one of claims 16 to 19, wherein the reagent comprises a second enzymatic HbAlc reagent, which comprises the compound B at a concentration of 1-50 g/L, preferably 15-35 g/L, and more preferably 15-25 g/L.

**22.** The reagent according to claim 20, wherein the reagent comprises a second enzymatic HbAlc reagent, which comprises the compound B at a concentration of 1-50 g/L, preferably 15-35 g/L, and more preferably 15-25 g/L.

**23.** The reagent according to claim 20 or 22, wherein the first enzymatic HbA1c reagent further comprises 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

**24.** The reagent according to claim 21 or 22, wherein the second enzymatic HbAlc reagent further comprises 10-200 KU/L of a fructosyl peptide oxidase and 5-100 KU/L of a peroxidase, and optionally a buffer, a preservative, and a protectant.

**25.** The reagent according to any one of claims 16 to 24, further comprising a sample treatment solution containing 1-10 mmol/L of sodium nitrite and optionally a preservative.

**26.** The reagent according to claim 25, wherein when the reagent is the reagent according to claim 20, the reagent further comprises a fourth enzymatic HbAlc reagent, which comprises 10-200 KU/L of a fructosyl peptide oxidase and 5-100 KU/L of a peroxidase, and optionally a buffer, a preservative, and a protectant.

**27.** The reagent according to claim 25, wherein when the reagent is the reagent according to claim 21, the reagent further comprises a third enzymatic HbAlc reagent, the third enzymatic HbAlc reagent comprising 200-2000 KU/L of an endo-peptide or exo-peptide protease, 0.1-100 mmol/L of a protease activator, 0.01-10 mmol/L of a color developer, and 0.1-5 g/L of an oxidant, and optionally a buffer, a preservative, and a protectant.

**28.** The reagent according to any one of claims 25 to 27, further comprising an Hb test reagent.

**29.** A reagent for detecting glycated hemoglobin in a blood sample, comprising:

an enzymatic sample treatment reagent, which comprises 1-10 mmol/L of sodium nitrite;
a first enzymatic HbAlc reagent, which comprises 200-2000 KU/L of an endo-peptide or exo-peptide protease, 0.1-100 mmol/L of a protease activator, 0.01-10 mmol/L of a color developer, and 0.1-5 g/L of an oxidant; and
a second enzymatic HbAlc reagent, which comprises 10-200 KU/L of a fructosyl peptide oxidase and 5-100 KU/L of a peroxidase,
wherein the first enzymatic HbAlc reagent and/or the second enzymatic HbAlc reagent further comprises 1-50 g/L of an amphiphilic compound,
wherein when the reagent is used to test the blood sample with a hemoglobin content 300 g/L or less, and a test result of glycated hemoglobin has linear consistency.

**30.** The reagent according to claim 29, wherein the amphiphilic compound is selected from at least one of compound A' and compound B, wherein the compound A' and the compound B are as defined according to any one of claims 16 to 19.

**31.** An enzymatic method for detecting glycated hemoglobin, comprising:
treating a blood sample using the reagent according to any one of claims 16 to 24.

**32.** The method according to claim 31, wherein a hemoglobin content in the blood sample is 300 g/L or less, 20-300 g/L, and preferably 20-200 g/L.

**33.** The method according to claim 31 or 32, wherein the method is a dual detection method or a non-dual detection method.

**34.** Use of compound A in a method for detecting glycated hemoglobin in a blood sample, wherein the compound is represented by the following formula A:

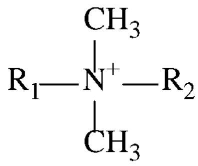

(A)

wherein

$R_1$ is selected from a C10-C20 alkyl and $-(CH_2)_{1-3}-N(H)C(O)-R_3$, wherein $R_3$ is selected from a C8-C14 alkyl; and $R_2$ is selected from $-(CH_2)_{1-3}COO^-$ and $-(CH_2)_{1-3}SO_3^-$, and

wherein a hemoglobin content in the blood sample is less than or equal to 60 g/L, preferably less than or equal to 40 g/L, and more preferably less than or equal to 30 g/L.

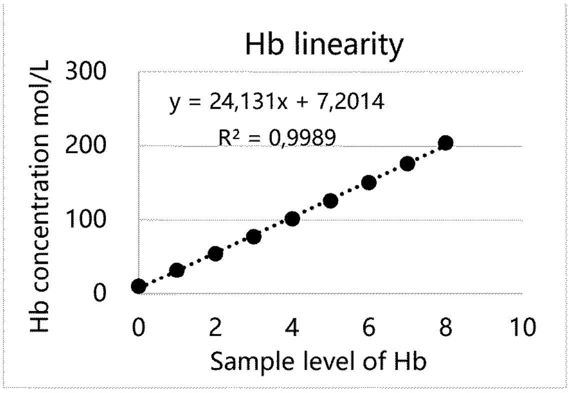
Hb linearity
Hb concentration mol/L
300
200
100
0
y = 24,131x + 7,2014
$R^2$ = 0,9989
0
2
4
6
8
10
Sample level of Hb

A

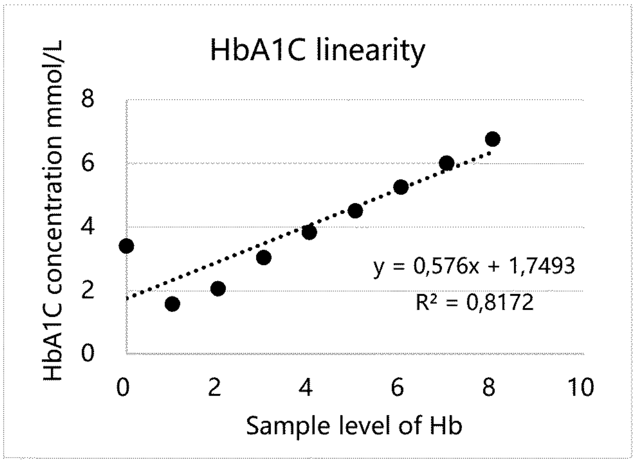
HbA1C linearity
HbA1C concentration mmol/L
8
6
4
2
0
y = 0,576x + 1,7493
$R^2$ = 0,8172
0
2
4
6
8
10
Sample level of Hb

B

FIG. 1

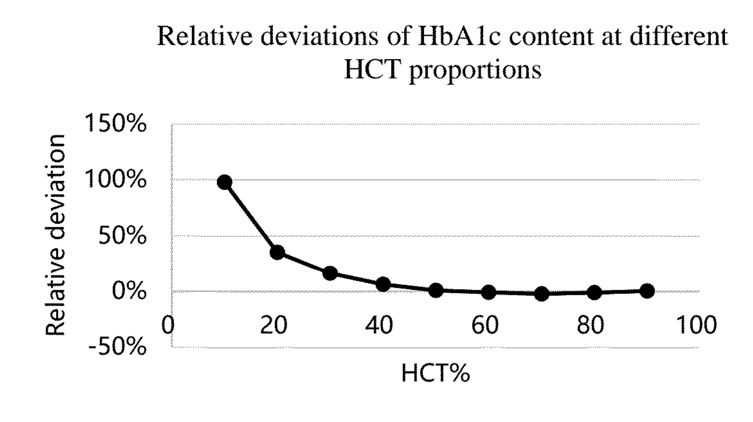
Relative deviations of HbA1c content at different HCT proportions
Relative deviation
150%
100%
50%
0%
-50%
0
20
40
60
80
100
HCT%

FIG. 2

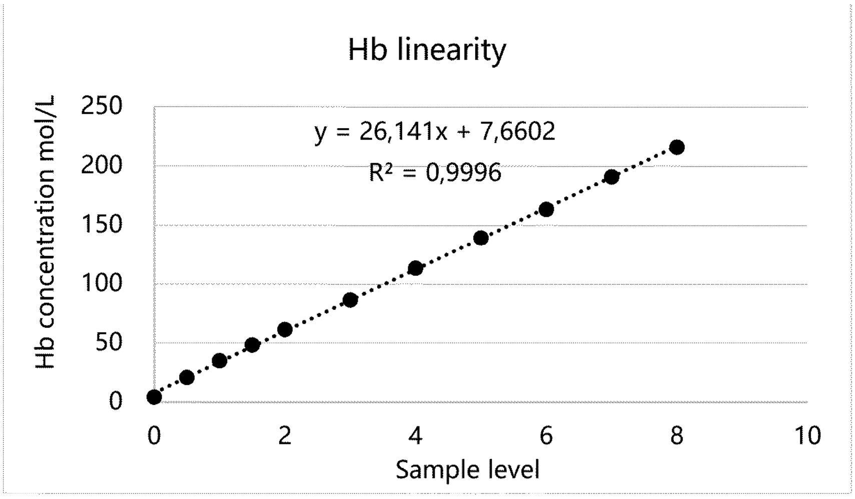
Hb linearity
y = 26,141x + 7,6602
R² = 0,9996
Hb concentration mol/L
250
200
150
100
50
0
0
2
4
6
8
10
Sample level

A

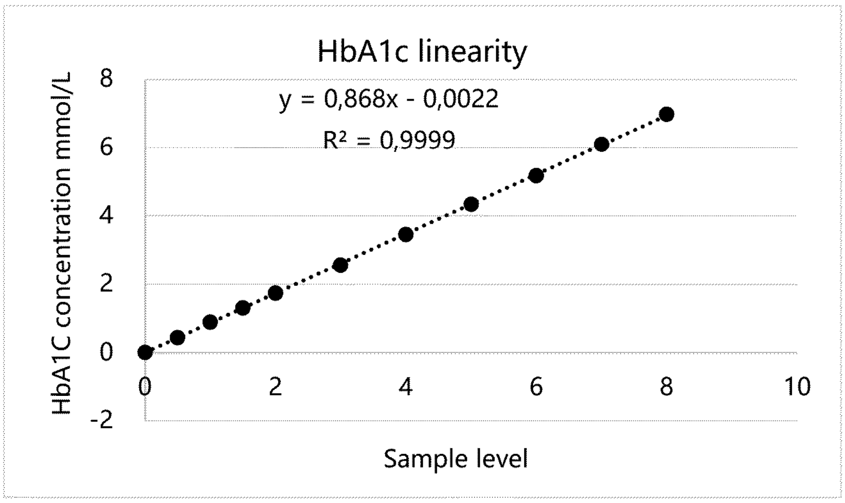
HbA1c linearity
y = 0,868x - 0,0022
R² = 0,9999
HbA1C concentration mmol/L
8
6
4
2
0
-2
0
2
4
6
8
10
Sample level

B

FIG. 3

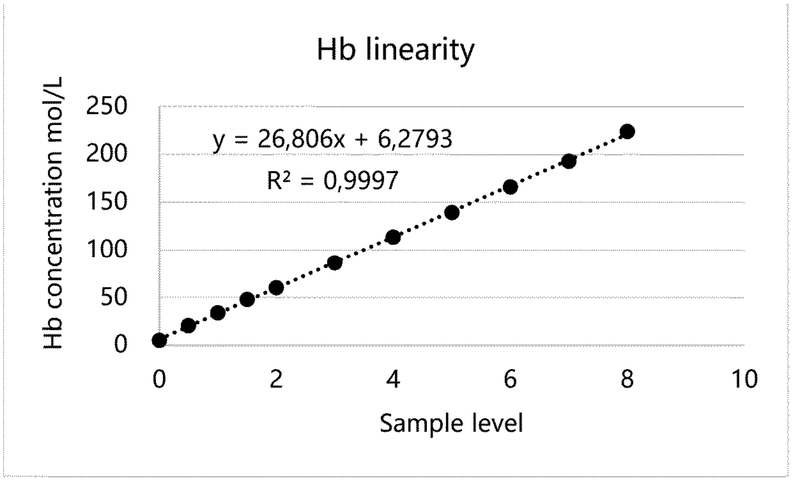
Hb linearity
Hb concentration mol/L
250
200
150
100
50
0
y = 26,806x + 6,2793
R² = 0,9997
0
2
4
6
8
10
Sample level

A

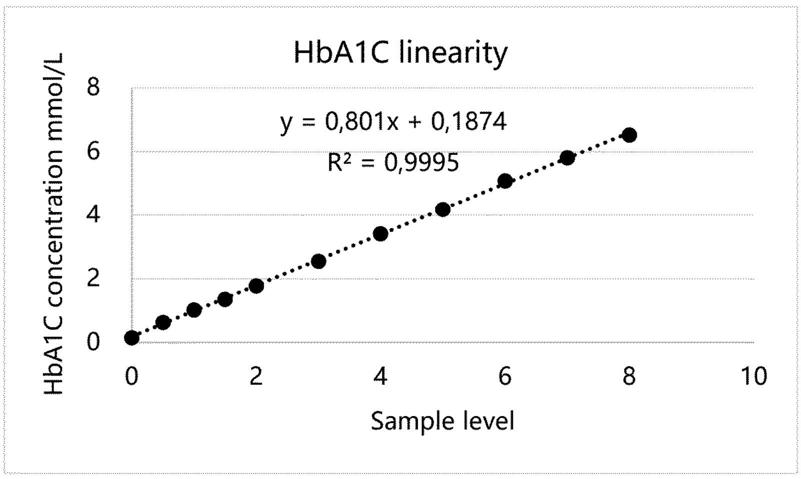
HbA1C linearity
HbA1C concentration mmol/L
8
6
4
2
0
y = 0,801x + 0,1874
R² = 0,9995
0
2
4
6
8
10
Sample level

B

FIG. 4

# INTERNATIONAL SEARCH REPORT

International application No.
**PCT/CN2023/142070**

## A. CLASSIFICATION OF SUBJECT MATTER

G01N 33/72(2006.01)i；G01N 33/53(2006.01)i；C12Q 1/28(2006.01)i

According to International Patent Classification (IPC) or to both national classification and IPC

## B. FIELDS SEARCHED

Minimum documentation searched (classification system followed by classification symbols)

IPC：G01N，C12Q

Documentation searched other than minimum documentation to the extent that such documents are included in the fields searched

Electronic data base consulted during the international search (name of data base and, where practicable, search terms used)

CNTXT&CNKI&ENTXTC: 糖化血红蛋白, 血红蛋白, 两性化合物, 两性表面活性剂, 甜菜碱, 磷脂酰胆碱, 磷酰胆碱
ENTXT&VEN&ISI: HbA1c, hemoglobin, hemoglobin A1c, glycohemoglobin, glycated haemoglobin, agglutination, zwitterionic detergent, phosphorylcholine

## C. DOCUMENTS CONSIDERED TO BE RELEVANT

| Category* | Citation of document, with indication, where appropriate, of the relevant passages | Relevant to claim No. |
|---|---|---|
| X | WO 0221142 A1 (WAKO PURE CHEMICAL INDUSTRIES, LTD.) 14 March 2002 (2002-03-14)<br>claims, description, pages 4-17, and embodiments 3-5 | 1-34 |
| X | CN 1449495 A (KYOWA MEDEX CO., LTD. et al.) 15 October 2003 (2003-10-15)<br>abstract, claims, and description, pages 6-12 | 1-15 |
| X | CN 106574933 A (DIASYS DIAGNOSTIC SYSTEMS GMBH) 19 April 2017 (2017-04-19)<br>abstract, claims, and description, pages 6-8 | 16-34 |
| A | CN 104105968 A (I-SENS, INC.) 15 October 2014 (2014-10-15)<br>entire document | 1-34 |
| A | CN 109680036 A (ARKRAY, INC.) 26 April 2019 (2019-04-26)<br>entire document | 1-34 |
| A | CN 103620052 A (KYOWA MEDEX CO., LTD.) 05 March 2014 (2014-03-05)<br>entire document | 1-34 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 April 2024** | **08 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/142070**

**C. DOCUMENTS CONSIDERED TO BE RELEVANT**

| Category* | Citation of document, with indication, where appropriate, of the relevant passages | Relevant to claim No. |
|---|---|---|
| A | CN 110023503 A (TOYOBO CO., LTD.) 16 July 2019 (2019-07-16) entire document | 1-34 |
| A | JP 2001033442 A (A & T: KK) 09 February 2001 (2001-02-09) entire document | 1-34 |
| A | US 2020200769 A1 (HITACHI CHEMICAL DIAGNOSTICS SYSTEMS CO., LTD.) 25 June 2020 (2020-06-25) entire document | 1-34 |

Form PCT/ISA/210 (second sheet) (July 2022)

**INTERNATIONAL SEARCH REPORT**
**Information on patent family members**

International application No.
**PCT/CN2023/142070**

| Patent document cited in search report | | | Publication date (day/month/year) | Patent family member(s) | | | Publication date (day/month/year) |
|---|---|---|---|---|---|---|---|
| WO | 0221142 | A1 | 14 March 2002 | AU | 8255001 | A | 22 March 2002 |
| | | | | JPWO | 2002021142 | A1 | 15 January 2004 |
| CN | 1449495 | A | 15 October 2003 | ATE | 438097 | T1 | 15 August 2009 |
| | | | | AU | 8021001 | A | 13 March 2002 |
| | | | | US | 2003166302 | A1 | 04 September 2003 |
| | | | | US | 7166476 | B2 | 23 January 2007 |
| | | | | KR | 20030029860 | A | 16 April 2003 |
| | | | | KR | 100778102 | B1 | 27 November 2007 |
| | | | | EP | 1314982 | A1 | 28 May 2003 |
| | | | | EP | 1314982 | A4 | 28 June 2006 |
| | | | | EP | 1314982 | B1 | 29 July 2009 |
| | | | | WO | 0218953 | A1 | 07 March 2002 |
| | | | | DE | 60139408 | D1 | 10 September 2009 |
| | | | | CA | 2420770 | A1 | 07 March 2002 |
| | | | | CA | 2420770 | C | 25 May 2010 |
| | | | | JP | 4733335 | B2 | 27 July 2011 |
| CN | 106574933 | A | 19 April 2017 | WO | 2015169511 | A2 | 12 November 2015 |
| | | | | WO | 2015169511 | A3 | 30 December 2015 |
| | | | | BR | 112016019822 | A2 | 15 August 2017 |
| | | | | BR | 112016019822 | A8 | 06 July 2021 |
| | | | | BR | 112016019822 | B1 | 14 November 2023 |
| | | | | EP | 3140661 | A2 | 15 March 2017 |
| | | | | EP | 3140661 | B1 | 15 August 2018 |
| | | | | JP | 2017515475 | A | 15 June 2017 |
| | | | | JP | 6636456 | B2 | 29 January 2020 |
| | | | | WO | 2015169862 | A1 | 12 November 2015 |
| | | | | KR | 20160147726 | A | 23 December 2016 |
| | | | | KR | 102280411 | B1 | 23 July 2021 |
| | | | | US | 2017058318 | A1 | 02 March 2017 |
| | | | | US | 10093959 | B2 | 09 October 2018 |
| | | | | US | 2017191112 | A1 | 06 July 2017 |
| | | | | US | 10202632 | B2 | 12 February 2019 |
| | | | | RU | 2016147532 | A | 06 June 2018 |
| | | | | RU | 2016147532 | A3 | 06 November 2018 |
| | | | | RU | 2683773 | C2 | 02 April 2019 |
| | | | | EP | 3140660 | A1 | 15 March 2017 |
| | | | | EP | 3140660 | B1 | 11 July 2018 |
| CN | 104105968 | A | 15 October 2014 | KR | 101207418 | B1 | 04 December 2012 |
| | | | | US | 2014370539 | A1 | 18 December 2014 |
| | | | | US | 9435792 | B2 | 06 September 2016 |
| | | | | EP | 2813854 | A4 | 17 December 2014 |
| | | | | EP | 2813854 | A1 | 07 September 2016 |
| | | | | WO | 2013118960 | A1 | 15 August 2013 |
| | | | | JP | 2015509361 | A | 30 March 2015 |
| | | | | JP | 5829766 | B2 | 09 December 2015 |
| CN | 109680036 | A | 26 April 2019 | EP | 3461908 | A1 | 03 April 2019 |
| | | | | EP | 3461908 | B1 | 30 August 2023 |
| | | | | US | 2019137509 | A1 | 09 May 2019 |
| | | | | US | 11313863 | B2 | 26 April 2022 |

Form PCT/ISA/210 (patent family annex) (July 2022)

**INTERNATIONAL SEARCH REPORT**
**Information on patent family members**

International application No.
**PCT/CN2023/142070**

| Patent document cited in search report | | | Publication date (day/month/year) | Patent family member(s) | | | Publication date (day/month/year) |
|---|---|---|---|---|---|---|---|
| CN | 103620052 | A | 05 March 2014 | JP | 2017023155 | A | 02 February 2017 |
| | | | | BR | 112013030886 | A2 | 21 March 2017 |
| | | | | EP | 2722396 | A1 | 23 April 2014 |
| | | | | EP | 2722396 | A4 | 11 March 2015 |
| | | | | US | 2015132786 | A1 | 14 May 2015 |
| | | | | CA | 2835729 | A1 | 29 November 2013 |
| | | | | WO | 2012173185 | A1 | 20 December 2012 |
| | | | | CA | 2839372 | A1 | 20 December 2012 |
| | | | | KR | 20140027383 | A | 06 March 2014 |
| | | | | JPWO | 2012173185 | A1 | 23 February 2015 |
| | | | | JP | 6236318 | B2 | 22 November 2017 |
| CN | 110023503 | A | 16 July 2019 | JPWO | 2018101389 | A1 | 24 October 2019 |
| | | | | JP | 7056577 | B2 | 19 April 2022 |
| | | | | WO | 2018101389 | A1 | 07 June 2018 |
| | | | | EP | 3550030 | A1 | 09 October 2019 |
| | | | | EP | 3550030 | A4 | 12 August 2020 |
| JP | 2001033442 | A | 09 February 2001 | None | | | |
| US | 2020200769 | A1 | 25 June 2020 | JPWO | 2018221446 | A1 | 02 April 2020 |
| | | | | JP | 7020484 | B2 | 16 February 2022 |
| | | | | WO | 2018221446 | A1 | 06 December 2018 |
| | | | | EP | 3636767 | A1 | 15 April 2020 |
| | | | | EP | 3636767 | A4 | 03 March 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211691520